# FASCICULE DE BREVET EUROPEEN

(11) **EP 0 518 731 B1**
(45) Date de publication et mention de la délivrance du brevet: **26.08.1998**
(21) Numéro de dépôt: 92401518.3
(22) Date de dépôt: 03.06.1992
(51) Int. Cl.: C07D 417/12, C07D 513/04, C07D 277/40, A61K 31/425, C07D 417/04, C07D 417/14, A61K 31/445

(54) **Dérivés de 2-(indol-2-yl-carbonylamino)-thiazoles,leur préparation et compositions pharmaceutiques en contenant**
2-(Indol-2-yl-carbonylamino)-thiazolderivate, ihre Herstellung und diese enthaltende pharmazeutische Zubereitungen
2-(Indol-2-yl-carbonylamino)-thiazole derivatives, their preparation and pharmaceutical compositions containing them

(30) Priorité: 05.06.1991 FR 9106814
(43) Date de publication de la demande: 16.12.1992
(73) Titulaire: SANOFI, 75008 Paris (FR)
(72) Inventeur: Boigegrain, Robert, F-34820 Assas (FR); Brodin, Roger, F-34070 Montpellier (FR); Gully, Danielle, F-31600 Saubens (FR); Molimard, Jean-Charles, F-34980 Saint Gely du Gesc (FR); Olliero, Dominique, F-34100 Montpellier (FR)
(74) Mandataire: Polus, Camille

(56) Documents cités:
- EP-A- 208 510
- EP-A- 308 885
- EP-A- 348 523
- EP-A- 432 040
- DE-A- 3 705 934
- US-A- 3 210 368

## Description

La présente invention concerne des dérivés hétérocycliques, qui interagissent avec le récepteur de la cholécystokinine et de la gastrine.

La cholécystokinine (CCK), est une hormone polypeptidique présente in vivo sous plusieurs formes comportant de 8 à 39 aminoacides. Elle a de nombreuses activités physiologiques sur les voies biliaires, le tractus gastrointestinal comme sur les systèmes nerveux central et périphérique et on peut se référer à l'article de J. E. Morley dans Life Sciences, 1982, 30, p. 479-493, qui fait une revue détaillée de ses propriétés. Deux populations différentes de récepteurs de la CCK ont été mises en évidence à l'aide d'antagonistes spécifiques ; ceux de type A présents en particulier dans le pancréas, la vésicule biliaire et certaines zones du système nerveux central, tandis que ceux du type B se trouvent surtout dans le système nerveux central.

La gastrine est une hormone polypeptidique qui agit notamment sur la sécrétion acide de l'estomac ; ses 5 aminoacides C-terminaux sont identiques à ceux de la CCK.

On a déjà décrit des composés antagonistes de la gastrine et/ou de la CCK, notamment le proglumide, le p-chlo- robenzoyl-L-tryptophane, ou plus récemment, des dérivés de benzodiazépines antagonistes spécifiques soit des récepteurs CCK A, tel que le 3S(-)-N-2-[1-méthyl-2-oxo-5-phényl-2,3-dihydro-1H-1,4-benzodiazépine-3-yl]-2-indole- carboxamide (J. Med. Chem., 1988, 31, 2235-46), soit des récepteurs CCK B, tel que la 3R(+)-N-[i-méthyl-2-oxo-5- phényl-2,3-dihydro-1H-1,4-benzodiazépine-3-yl] N'-[3-méthylphényl]urée (Eur. J. Pharmacology, 1989, 162, 273-280).

Par ailleurs, des dérivés du thiazole de formule : 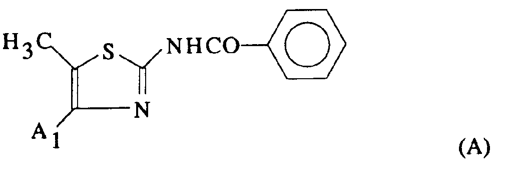 dans laquelle A₁ représente un 2,4-diméthoxyphényle ; un 2,3,4-triméthoxyphényle ou un groupe hétérocyclique tel qu'un 3,4-dihydro-7-méthoxy-2,2,8-triméthylbenzopyrane-1-2 H-6-yl ou un 3,4-dihydro-7-méthoxy-2,2-diméthylben- zopyrane-1-yl-2H-6-yl sont décrits dans Indian J. Chem., Sect. B, 1988, 27 (B) 7, 629-32 comme possédant des propriétés bactéricides ou fongicides.

D'autres dérivés du thiazole de formule : 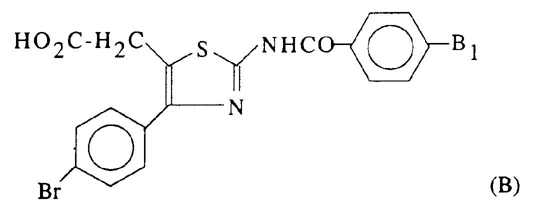 dans laquelle B₁ représente l'hydrogène ou un atome de brome sont décrits dans Chem. Pharm. Bull., 1977, 25 (9), 2292-9 comme possédant des propriétés antiinflammatoires.

D'autres dérivés du thiazole de formule : 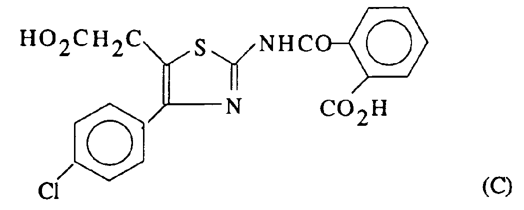 possèdent des propriétés immunostimulatrices et antiinflammatoires et sont décrits dans Arch. Immunol. Ther. Exp., 1978, 26 (1-6), 921-9.

Des dérivés de 4-quinolinecarboxamides de formule : 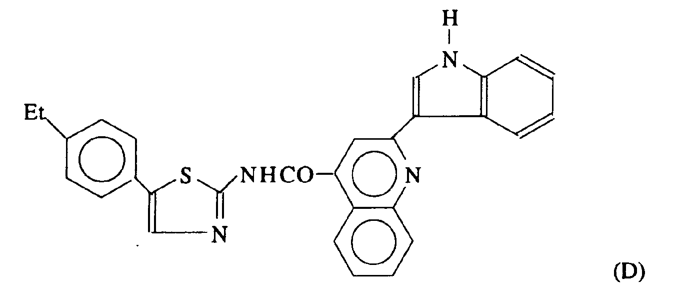 sont cités dans Chem. Abst. 112 (13), 115 589 x comme possédant des propriétés bactéridices et désinfectantes.

D'autres dérivés du thiazole ayant des propriétés anti-CCK sont décrits dans EP-A-0 432 040. EP-A-432 040 concerne des antagonistes de la chlocystokinine de formule 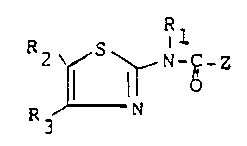 dans laquelle le substituant R₂, en position 5 du noyau acylaminothiazole représente un atome d'hydrogène ou un groupe alkyle en Ci-C₄. Ce document fait partie de l'état de la technique au sens de l'article 54(3) CBE.

US-A-3 210 368 concerne des antagonistes de la norepinéphrine de formule :

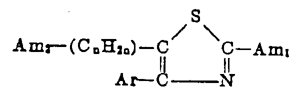

Ces composés ne présentent pas de groupe acylamino en position 2 du noyau thiazole.

EP-A-348 523 a trait a des antagonistes de la cholecystokinine et de la gastrine qui sont des thiénodiazépines.

Les composés décrits dans EP-A-308 885 ont pour formule générale 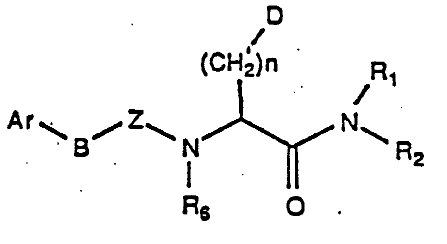 et fonctionnent en tant qu'antagonistes de la cholecystokinine.

Dans ces composés Ar peut représenter thiazolyle mais 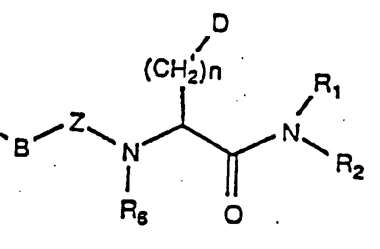 ne représente pas un groupe heteroarylcarbonylamino.

EP-A-208 510 propose de nouveaux agents anti-inflammatoires de formule générale 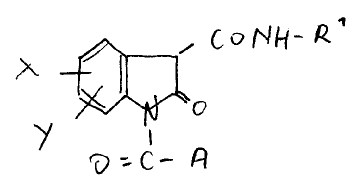 qui ne peuvent pas être assimilés à des thiazoles substitués en position 2 par un groupe indolylcarbonylamino, bien que R₁ puisse représenter thiazolyle.

DE-A-37 05 934 a trait à des composés de formule 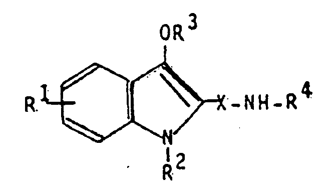 présentant une bonne affinité pour les récepteurs de la benzodiazépine.

Dans ces composés R⁴ peut représenter thiazolyle non-substitué.

Les composés selon l'invention sont des dérivés hétérocycliques de 2-aminothiazoles de formule (I) : 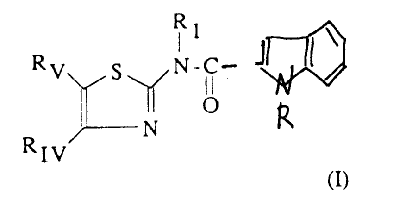 dans laquelle
- R₁ représente l'atome d'hydrogène ; un groupe alkyle en C₁ à C₄ ou un groupe aminoalkylène de formule -Z₁-NR₄R₅ dans laquelle Z₁ représente un alkylène en C₂ à C₄ et R₄ et R₅ représentent indépendamment H ou un alkyle en C₁ à C₄
- R_{IV} représente un groupe phényle non substitué ou portant un ou plusieurs substituants choisis parmi les atomes d'halogène, les groupes (Ci-C₆) alkyle, et alcoxy en C₁ à C_{3,} ou R_{IV} et Rᵥ considérés ensemble représentent le groupe : 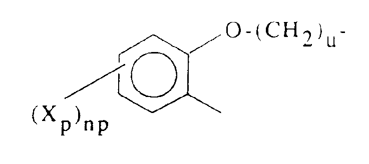 fixé par le carbone du phényle en position 4 du noyau thiazolyle et dans lequel u vaut 1 à 3, portant éventuellement un ou plusieurs (np) substituants Xp, identiques ou différents choisis parmi les atomes d'halogène, les groupes alkyle et alcoxy en C₁ à C_{3,} les groupes nitro et trifluorométhyle, np valant de 0 à 3 ;
- Rᵥ représente un groupe -(CH₂)ₘ-X dans lequel m est 0 à 5 et X représente
   un atome d'halogène, un hydroxyle, un cycloalkyle en C₃ à C₇, un phényle qui peut être substitué par un des groupes choisis parmi les atomes d'halogène, les groupes alkyles ou alcoxy en C₁ à C₃, nitro, amino, hydroxy ou trifluorométhyle ;
   un groupe choisi parmi -COOH ; -COOX₁ ; -O-COX₁ ; -SCOX₁ ; (O)_{q}-S-X₁ avec q = 0,1 ou 2 ; -O-COOX₁ ; 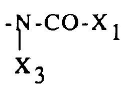 avec X₃ = H ou alkyle en C₁ à C₃ ; 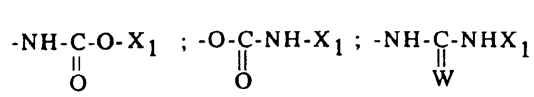 avec W=O ou S ; 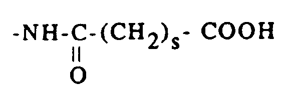 avec s = 2,3,4
   dans lequel X₁ représente un alkyle en C₁ à C₅ ; un phényle qui peut être substitué par un ou des groupes choisis parmi les atomes d'halogène, les groupes alkyles ou alcoxy en C₁ à C_{3,} nitro, amino, hydroxy, trifluorométhyle ; un groupe adamantyle ;
   un groupe choisi parmi -CONX₁X₂ ; -NX₁X₂ ; dans lequel X₁ représente l'hydrogène, un alkyle en C₁ à C₃ ou un phényle non substitué ou substitué par un ou des groupes choisis parmi les atomes d'halogène, les groupes alkyles ou alcoxy en Ci à C_{3,} nitro, amino, hydroxy, trifluorométhyle et X₂ représente un atome d'hydrogène, un alkyle en C₁ à C₃, ou bien, X₁ et X₂ constituent avec l'atome d'azote auquel ils sont liés un hétérocycle choisi parmi la pyrrolidine ou la pipéridine non substitué ou substitué par un groupe oxo ou par un groupe hydroxyle, ce dernier étant non substitué ou substitué par un acyle, par un groupe -COOX₁ ou - CONX₁X₂ ;
   ou encore Rᵥ représente un alcoxy en C₁-C₅ ; un groupe hydroxyle ; une amine cyclique à 5 ou 6 chaînons non substituée ou substituée par un groupe oxo ou un groupe hydroxyle ; un groupe pipérazinyle non substitué ou N-substitué par un groupe -COOAlk dans lequel Alk représente un alkyle en C₁-C₅ ; un groupe acide carboxylique, un groupe -NX₂X₄ avec X₄ = H ou X₄ = -(CH₂)ₜ-X₅, avec t égal à 2, 3 ou 4 et X₅ représente 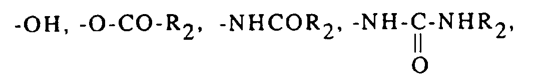
   dans lequel R₂ représente un alkyle C₁-C₆ ; ou un groupe -NR₂R₃ avec R₂ ou R₃ représentant indépendamment H, alkyle en Ci à C₆, un groupe phényle non substitué ou substitué par un ou plusieurs substituants choisis parmi les atomes d'halogène, un groupe alkyle en Ci à C₃, un groupe alcoxy en Ci à C₃ ou R₂ et R₃ constituent avec l'atome d'azote auquel ils sont liés un hétérocycle à 5 ou 6 chainons ;
- R représente H, (Ci-C₄) alkyle, carboxyalkylène -Z₄-COOR₁₀ dans laquelle Z₄ représente (C₁-C₄) alkylène et R₁₀ est H, benzyle ou (C₁-C₆) alkyle ; carbamoyalkylène -Z₅-CONR₁₁ R₁₂ dans laquelle Z₅ représente (C₁-C₄) alkylène et R₁₁ et R₁₂ représentent indépendamment H, (Ci-C₆) alkyle, ou forment avec N un hétérocycle saturé comme morpholino ou pipéridino ; acyle COR₁₃ avec R₁₃ représente (C₁-C₄) alkyle ou phényle ; alcoxycarbonyle -COOR₁₄ avec R₁₄ étant tert-butyle ou benzyle;

ainsi que les sels d'addition de ces composés avec des acides et des bases minérales ou organiques ; les sels non toxiques pharmacetiquement acceptables sont préparés mais d'autres sels utilisables pour isoler ou purifier les composés de formule (I) sont aussi un objet de l'invention.

Les groupes alkyle, alkylène, alcoxy et thioalcoxy peuvent être linéaires ou ramifiés.

Parmi les composés de formule (I), on préfère ceux dans lesquels R₁ représente l'hydrogène ; parmi les groupes R_{IV,} on préfère le phényle.

La présente invention a également pour objet la préparation des composés de formule (I) qui sont préparés par condensation d'un aminothiazole de formule (II) : 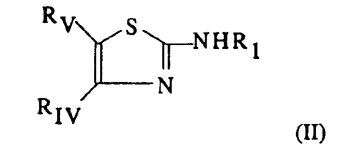 dans laquelle R₁, R_{IV} et Rᵥ ont les significations indiquées pour (I), dans les conditions habituelles d'acylation d'une fonction amine, avec un acide de formule 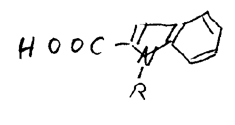 dans lequel les fonctions sensibles ont été protégées, et R₁, R_{V,} R_{IV} ont la même signification que dans la formule (I), ou avec une forme activée de l'acide telle qu'un halogénure d'acide, un anhydride d'acide, et de préférence un anhydride mixte comme un anhydride carbonique, ou un ester acitvé, obtenu avec les réactifs couramment utilisés en synthèse peptidique.

Les composés (II) peuvent être protégés ; R°₁ représente alors les mêmes substituants que R₁ dans lequel le groupe amino présent est N-protégé, R_{Va} représente les mêmes substituants que R_{V} dans lesquels les groupes hydroxy ou amino sont 0- et N-protégés.

Lorsque les fonctions ont été protégées, on effectue après la condensation la réaction de déprotection convenable, si nécessaire.

De nombreux aminothiazoles de formule (II) sont connus.

Les aminothiazoles nouveaux peuvent être préparés selon l'un des procédés décrits, notamment dans Bull. Soc. Chim. (C) 1963, 2498-2503.

De façon générale on fera réagir une thiourée avec une cétone alpha-halogénée, et de préférence alpha-bromée, selon le schéma réactionnel suivant :

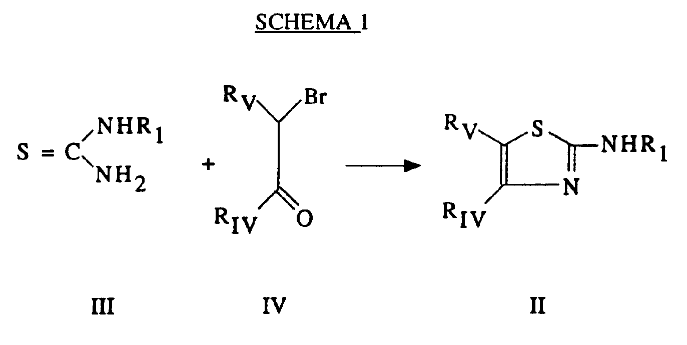

R₁, Rivet Rᵥ ayant la même signification que dans la formule (II).

La préparation de divers composés (II) dans lesquels R₁ représente un groupe aminoalkyle est décrite dans EP-A-0 283 390.

Les cétones alpha-halogénées et les thiourées peuvent être préparées par des procédés dont les principes sont décrits dans les ouvrages généraux ; ainsi les cétones alpha-bromées (IV) peuvent être préparées par action sur RᵥCH₂COR_{Iv} du brome en milieu acide acétique ou du bromure cuivrique dans un solvant organique tel que l'acétate d'éthyle, un solvant chloré ou leurs mélanges. Les cétones aromatiques de départ sont préparées en général par réaction de Friedel et Crafts, tandis que les méthylcétones aliphatiques peuvent être préparées par action du diazométhane sur les chlorures d'acides carboxyliques convenables suivie de l'hydrolyse de la diazocétone correspondante.

Les cétones aromatiques alpha-chlorées peuvent être préparées par réaction de Friedel et Crafts avec le chlorure d'acide alpha-chloré convenable.

Lorsque Rᵥ représente un groupe ester (CH₂)ₘ-COOX₁ les dérivés du thiazole correspondant de formule (V) ci-dessous dans lesquels R_{IV,} X₁ et m sont tels que définis pour (I) sont connus ou préparés selon des méthodes connues en faisant réagir un alpha-bromoacétoacide ou un alpha-bromocétoester sur la thiourée selon le schéma suivant :

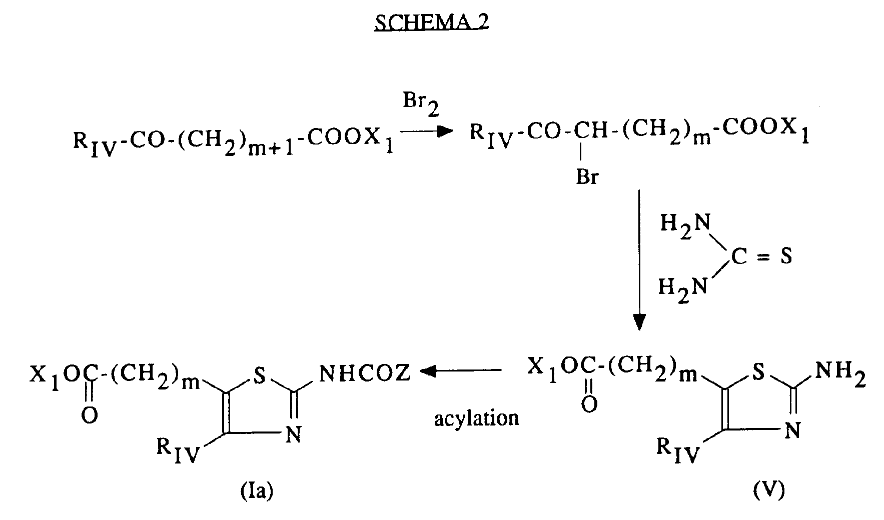

Selon la valeur du substituant R_{V}, les méthodes suivantes de préparation sont utilisées:
a) - lorsque Rᵥ représente un groupe -(CH₂)ₘ-OH, le dérivé du 2-aminothiazole correspondant de formule (VI) ci-dessous dans laquelle m est tel que défini pour (I) peut être préparé à partir des esters (V) précédents par réduction avec un hydrure alcalin tel que par exemple l'hydrure de lithium aluminium dans un solvant aprotique tel que par exemple le tétrahydrofurane pour conduire à l'aminoalcool de formule: 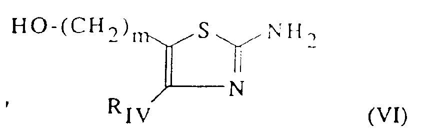 l'acylation de (VI) par 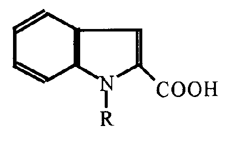 conduit au composé (Ib) de formule : 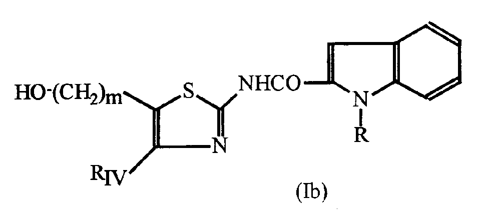 dans lequels m, R et R_{IV} sont tels que définis pour (I),
b) - lorsque Rᵥ représente un groupe ester -(CH₂)ₘ-O-CO-X₁ ou thioester -(CH₂)ₘ-S-COX₁ ou (O)q-S-X₁ dans lesquels m, X₁ et q sont tels que définis pour (I), les dérivés du 2-aminothiazole (VII), (Vllc) ou (Vlld) dans lequel les groupes R_{IV}, q, m, W et X₁ sont tels que définis pour (I) peuvent être préparés
   - soit selon le schéma 3 suivant :

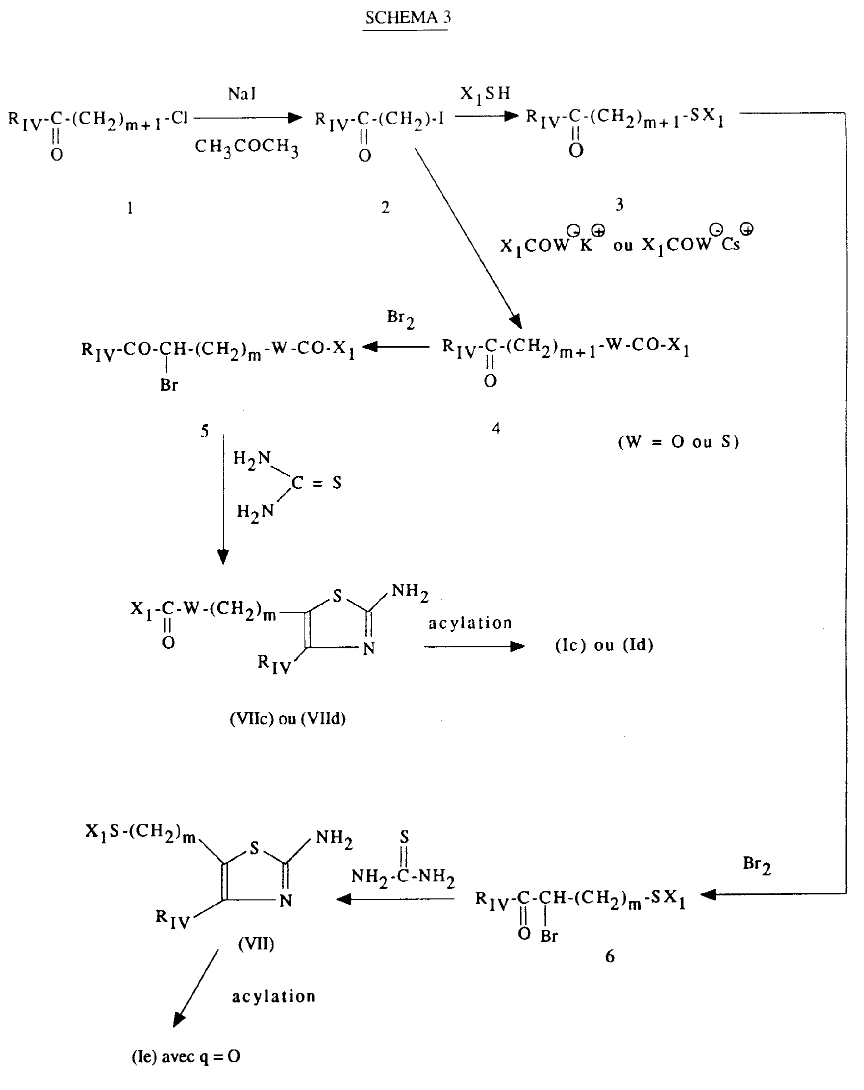

   - soit à partir des alcools (VI) N-protégés tel que définis ci-dessus sur lesquels on fait réagir un chlorure d'acide tel que par exemple le chlorure d'acétyle dans un solvant tel que par exemple la pyridine pour obtenir les esters de formule : 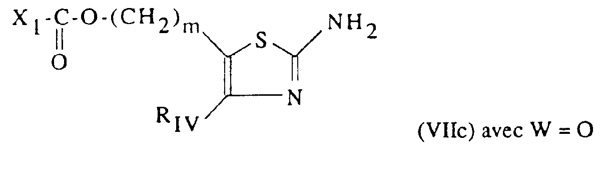 ou 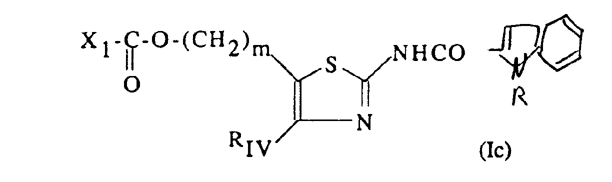 dans lesquels X₁, m, R et R_{IV} sont tels que définis ci-dessus pour (I).
c) - lorsque Rᵥ représente un carbamate -(CH₂)ₘ-O-CO-NHX₁ dans lequel m et X₁ sont tels que définis pour (I) les dérivés du thiazole selon l'invention sont préparés à partir des composés hydroxylés (Ib) correspondants, par action d'un isocyanate de formule X₁-N = C = O, dans un solvant aprotique tel que par exemple le tétranydrofurane ou le dichlorométhane à une température comprise entre 20°C et 100°C pour conduire au composé (If) de formule : 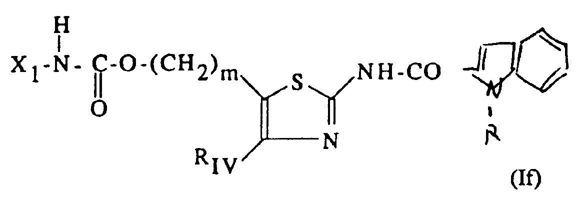 dans laquelle X₁, m, R et R_{IV} sont tels que définis pour (I).
d) - lorsque Rᵥ représente un amide -(CH₂)ₘ-CONX₁X₂ dans lequel m, X₁ et X₂ sont tels que définis pour (1), les thiazoles selon l'invention sont préparés par réaction de l'amine HNX₁X₂ sur l'ester correspondant de formule (V) ou (la) en présence ou non d'un solvant tel qu'un alcanol à une température comprise entre 20 et 120°C ; la réaction peut également être effectuée en tube scellé en fonction si l'amine est volatile pour conduire au composé (VIII) ou (Ig) de formule : 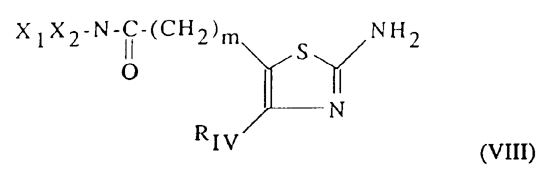 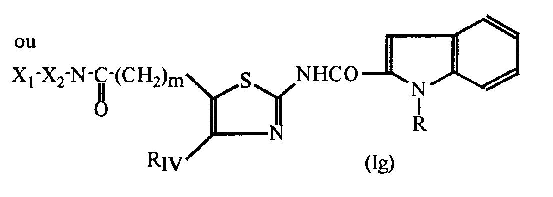 dans lesquels X₁, X₂, m, R et R_{IV} sont tels que définis pour (I).
e) - lorsque Rᵥ représente un groupe aminé -(CH₂)ₘ-NX₁X₂, les dérivés du thiazole selon l'invention sont préparés, par exemple, par réduction des amides précédemment décrits de formule (VIII) par réduction par un hydrure alcalin tel que par exemple l'hydrure de lithium aluminium dans un solvant tel que le tétrahydrofurane à une température comprise entre 20°C et la température d'ébullition du solvant pour conduire au composé de formule : 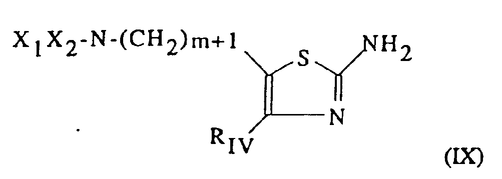 l'acylation de (IX) par 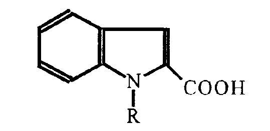 conduit au composé (Ih) de formule : 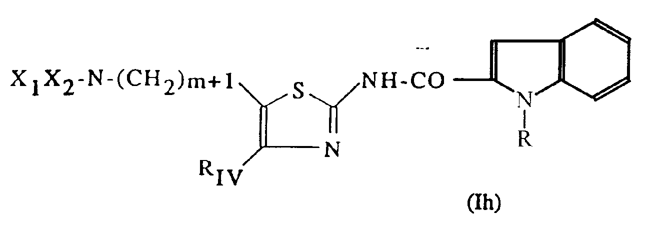 dans laquelle X₁, X₂, m, Rivet Z sont tels que définis pour (I),
f) - lorsque Rᵥ représente un carbonate -(CH₂)ₘ-O-COOX₁, dans lequel m et X₁ sont tels que définis pour (I), les thiazoles selon l'invention sont préparés à partir des alcools (Ib) en les mettant en réaction avec un chloroformiate 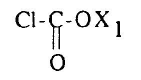 en présence d'une base telle que la triéthylamine ou la pyridine pour conduire au composé (li) de formule : 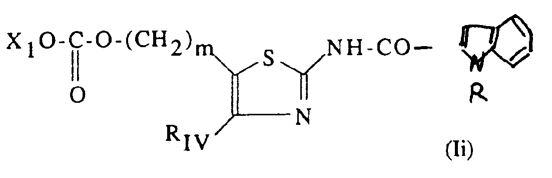 dans laquelle X₁, m, R_{IV} et R sont tels que définis pour (1),
g) - lorsque Rivet R_{V} considérés ensemble représentent le groupe : 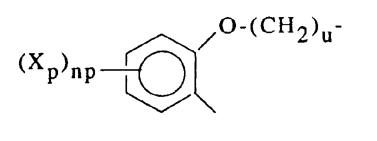 dans lequel (Xₚ)ₙₚ et u sont tels que définis pour (I), fixé par le carbone du phényle en position 4 du noyau thiazole ; par exemple, l'intermédiaire 4-bromo-2 H-dihydro-3,4-[1]-benzoxépine-5-one de formule : 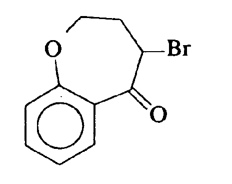 est préparé selon G. Fontaine et al., C.R. Acad. Sci., 1965, 258, 4583 ; la 2-amino-4,5-dihydro-[5,4-d]-thia- zolo-[1]-benzoxépine de formule : 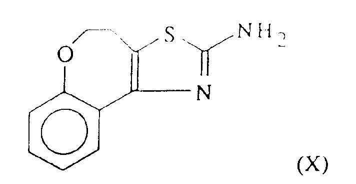 est préparée par cyclisation avec la thiourée selon le procédé habituel décrit précédemment puis acylé pour conduire au composé de formule : 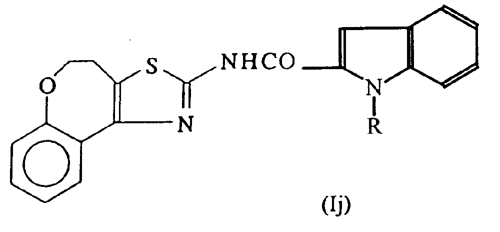 dans lequel R est tel que défini pour (I),
h) - lorsque Rᵥ représente un groupe amino -NX₂X₄ dans lequel X₂ et X₄ sont tels que définis pour (1), le dérivé du thiazole selon l'invention peut être préparé à partir du 2-amino-5-bromothiazole de formule : 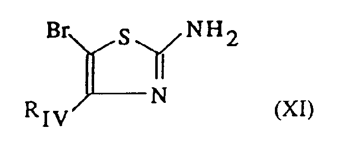 qui est préparé selon J. Chem. Soc., 1947, 114, peur être ensuite :
   - soit acylé, par exemple, par un dérivé de 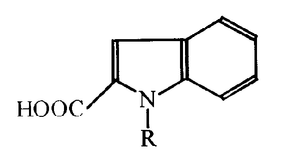 en présence de BOP et d'une base telle que la triéthylamine puis ce dérivé bromé obtenu de formule : 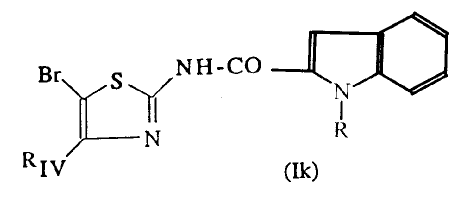 dans lequel Rivet R sont tels que définis pour (I), est substitué par un amine HNX₂X₄ dans un alcanol à une température comprise entre 20°C et la température d'ébullition du solvant pour conduire au composé de formule : 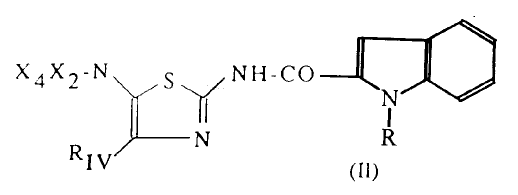 dans laquelle X₂, X₄, R_{IV} et R sont tels que définis pour (I),
   - soit substitué par l'amine HNX₂X₄ puis acylé en position 2 du thiazole, les deux réactions se faisant dans des conditions identiques à celles décrites ci-dessus,
i) - lorsque Rᵥ représente un groupe -(CH₂)ₘ-X dans lequel m = 0 et X représente un groupe alcoxy en C₁-C₅, le 2-aminothiazole correspondant est préparé à partir de la 2-bromo-2-alcoxy-1-phényléthanone éventuellement substituée sur le phényle pour conduire au produit de formule : 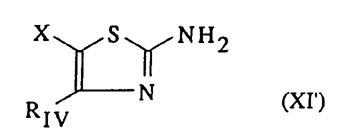 dans lequel R_{IV} est tel que défini précédemment et X représente un alcoxy en C₁-C₅, qui est ensuite acylé comme indiqué ci-dessus pour conduire aux composés (lm) de formule : 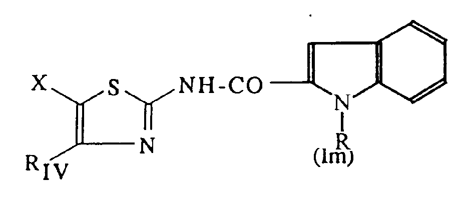 dans lequel Rivet R sont tels que définis pour (I) et X est tel que défini ci-dessus pour (XI'), ou un de leurs sels.

Les composés de formule (XI') sont des intermédiaires nouveaux faisant partie de l'invention.

Certains des acides 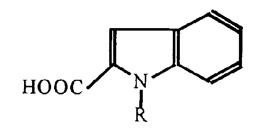 sont connus et même commerciaux; les autres sont préparés en utilisant les méthodes connues pour des molécules analogues.

Ainsi les acides indolecarboxyliques de formule : 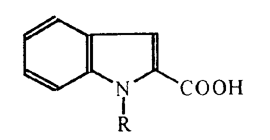 dans laquelle R représente un groupe alcoxycarbonylalkylène peuvent être préparés à partir des acides indolecarboxyliques commerciaux ou obtenus par des procédés classiques, en suivant le schéma réactionnel 4 ci-dessous.

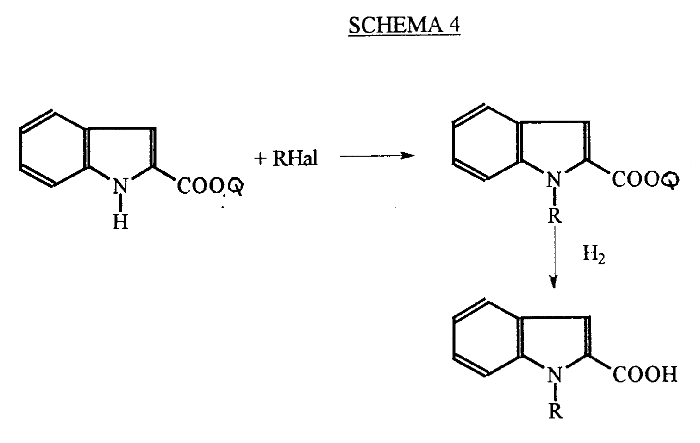

dans lequel Hal représente un atome d'halogène et Q représente le groupe benzyle.

Les esters benzyliques du schéma (4) sont préparés par action de l'acide correspondant sur l'alcool benzylique, en présence de l'un des agents d'activation de la fonction acide couramment utilisés en synthèse peptidique tel que :
- le 1,1'-carbonyldiimidazole pour lequel on pourra se référer à Synthesis 1982, p. 833,
- le N,N-dicyclohexylcarbodiimide en présence de 4-diméthylaminopyridine pour lequel on pourra se référer à J. Org. Chem. 1990, 55 (4), p. 1390,
- l'hexafluorophosphate de benzotriazolyloxy tris-(diméthylaminophosphonium) pour lequel on pourra se référer à Synthesis, 1977, p. 413.

La base mise en oeuvre lors de la fixation de Rg sur l'azote de l'ester benzylique est de préférence une base forte anhydre telle qu'un hydrure alcalin ; le mélange réactionnel est alors un solvant aprotique polaire, stable en présence d'une base forte, telle que le diméthylformamide ou le diméthoxyéthane ; la réaction est effectuée à une température comprise entre 15°C et 80°C environ.

L'élimination du groupe benzyle, après la N-alkylation est effectuée de façon classique par action d'au moins un équivalent d'hydrogène, en présence d'un catalyseur tel que le palladium sur du charbon, sur l'ester en solution dans un alcool ou le diméthylformamide, éventuellement sous une légère pression.

Par ailleurs, certains des acides ZCOOH sont peu stables ou portent une fonction qui pourrait réagir lors de la condensation avec l'aminothiazole et il est préférable de les mettre en oeuvre sous une forme protégée Z'COOH.

Ainsi les dérivés (I) dans lesquels R représente H peuvent être préparés à partir des composés obtenus par condensation de l'aminothiazole avec des composés de l'acide indolecarboxylique, de formule :

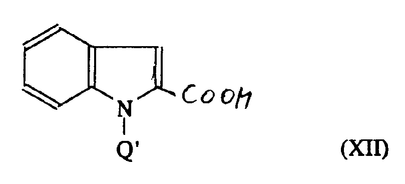

dans laquelle Q' représente un groupe habituellement utilisé pour la protection des groupes NH₂ dans les réactions de condensation des acides aminés, tel que 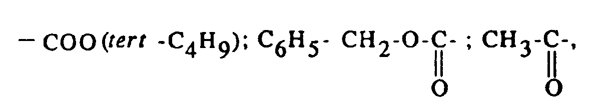 le groupe protecteur Q peut être éliminé du composé de formule : 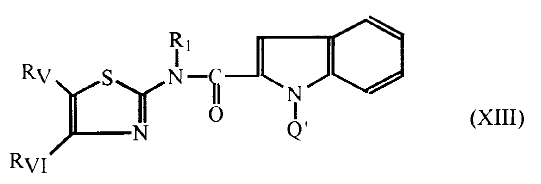 obtenu après la condensation avec le dérivé (II), par les méthodes de déprotections classiques.

Le BOC peut s'éliminer par pyrolyse, en l'absence de solvant à une température comprise entre 180 et 200° C.

Les acides indolecarboxyliques dans lesquels R est COOC(CH₃)₃ ou COOCH₂C₆Hₛ peuvent être préparés par action du dicarbonate de tert-butyle, du chloroformiate de benzyle sur l'acide dans lequel R = H, en présence d'une base telle que la triéthylamine ou la 4-diméthylaminopyridine, dans un solvant tel que l'acétonitrile ou le chlorure de méthylène.

Les acides dans lesquels R est un groupe acyle peuvent être préparés par action du chlorure ou de l'anhydride d'acide sur l'acide libre dans lequel R = H en présence d'un équivalent de triéthylamine et de 4-diméthylaminopyridine, par exemple dans le dichlorométhane.

Le chlorure d'acide peut être préparé, notamment, par action de SOCI₂ ou d'un mélange de POCI₃ et P₂0₅ sur l'acide correspondant, en général en l'absence de solvant et à la température de reflux du mélange réactionnel.

Parmi les esters activés de formule 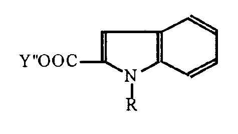 on peut préparer ceux dans lesquels Y" représente 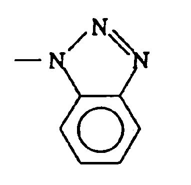 par action du 1-hydroxybenzotriazole sur l'acide en présence de dicyclohexylcarbodiimide selon le mode opératoire décrit dans J. Am. Chem. Soc. 1971, 93, 6318-6319 (1971), ou par action de l'hexafluorophosphate de 1-benzotriazo- lyloxytris(diméthylamino)phosphonium selon le mode opératoire décrit dans Synthesis, 1976, 751-752.

La condensation de l'aminothiazole (II) avec l'acide sous forme d'ester activé peut être effectuée dans un solvant dont la nature est choisie selon la solubilité des composés et le type d'activation de la fonction acide, de préférence en présence d'une base, par exemple une amine tertiaire telle que la triéthylamine ; la réaction est en général effectuée à une température comprise entre 0°C et 30°C.

Lorsque les composés de formule (I) comportent dans Z un groupe acide carboxylique, ceux-ci sont préparés par hydrolyse d'un ester de préférence en milieu basique, par exemple par action d'une base minérale, tel qu'un hydroxyle alcalin, en milieu hydroalcoolique ou hydrolyse acide dans le cas d'un ester tert-butylique.

Les sels d'addition des composés de formule (1) avec des acides ou des bases sont préparés de la façon habituelle par introduction de l'acide, ou de la base dans une solution du composé de formule (I). Le sel est isolé, selon ses caractéristiques de solubilité, après évaporation du solvant ou addition d'un non-solvant.

Les composés de formule (I) et leurs sels inhibent la fixation de la cholécystokinine, à ses récepteurs. Ils sont plus ou moins sélectifs des récepteurs de type A ou B, et des antagonistes de la gastrine plus ou moins puissants.

Leur affinité pour le récepteur CCK A a été déterminée, in vitro, en utilisant la méthode décrite dans ce qui suit, dont le principe est celui mentionné dans Life Sciences, 1985,37 (26), 2483-2490 ; elle consiste à déterminer le déplacement de la CCK 8S iodée de ses sites de fixation sur un homogénat de pancréas de rat : des quantités aliquotes de suspension membranaire pancréatique (100 µg de protéines par ml) dans un tampon TRIS, HCI (50mM), de pH = 7,4 contenant MgCl₂ (5 mM), bacitracine (0,1 mg/ml), fluorure d'acide méthylphénylméthane sulfonique (0,1 mg/ml), sont incubés 40 minutes à 25°C en présence de CCK 8S iodée (2000 Ci/mmole, soit 50 mM de concentration finale) et de concentrations croissantes de la substance à étudier ; la réaction est arrêtée au bout de 40 minutes par centrifugation. Après élimination du surnageant, la radioactivité du culot est mesurée. Par ailleurs, la fixation spécifique est déterminée en présence de CCK 8S à la concentration de 1 µM.

Dans ces conditions, la concentration inhibitrice de 50 % de la fixation (Cl₅₀) est pour les produits de l'invention inférieure à 10⁻⁷M, et pour un grand nombre de l'ordre de 10-¹⁰M.

Leur affinité pour les récepteurs CCK B a été déterminée en étudiant le déplacement de la CCK 8S iodée de ses sites de fixation spécifiques présents sur des homogénats de cortex de cobaye en utilisant le même mode opératoire que pour les récepteurs CCK A, mais pour une suspension membranaires à 600 µg de protéines/ml et avec un tampon HEPES (10mM) à pH 6,5 contenant NaCI (130 mM), Mg CI₂ (5mM), EDTA (1mM) et bacitracine (250 mg/ml)et l'incubation étant de 2 heures.

A la concentration de 10⁻⁵M tous les produits déplacent plus de 25 % de la CCK 8S marquée du récepteur B ; certains ont des Cl₅₀ de l'ordre de 10⁻⁹M.

L'affinité pour le récepteur de la gastrine des composés les plus spécifiques du récepteur CCK B a été étudiée, selon la méthode décrite dans ce qui suit, dont le principe est celui mentionné dans J. Receptor. Res., 1983,3 3 (5) 647-655 ; des aliquotes de glandes gastriques de cobaye dans un tampon à pH = 7,4 HEPES (24,5 mM) comprenant NaCI (98 mM), KCI (6 mM), NaH₂ P0₄ (2,5 mM), pyruvate (5 mM), CaCl₂ (0,5 mM), MgCI₂ (1 mM), glucose (11,5 mM), glu- tamine (1 mM), albumine bovine (0,4 g/100 ml) ont été incubée 90 minutes à 37°C dans un bain marie en présence de gastrine (2-17) iodée (2000 Ci/mmol ; 70 pM) et de concentrations croissantes des produits à étudier. La réaction a été arrêtée par centrifugation et la radioactivité du culot mesurée ; la fixation non spécifique a été déterminée en présence de gastrine (2-17) à 1 µM. Les composés de l'invention ont une Cl₅₀ comprise entre 10⁻⁵M et 10⁻⁹M.

On a aussi montré que les composés de l'invention ont une activité inhibitrice de celle de la CCK. Ceci a été mis en évidence in vitro par la mesure de l'inhibition par les produits à tester, de la sécrétion d'amylase par des acini de rats stimulée par la CCK 8S, selon une méthode analogue à celle décrite dans J. Biol. Chem., 1979, 254 (12), 5321-5327, mais avec des tissus pancréatiques de cobaye. Les composés ont une Cl₅₀ de 10⁻⁶ M à 10⁻⁹M.

Enfin, in vivo, chez la souris, les composés ayant une bonne affinité pour les récepteurs de la CCK A ont antago- nisé l'inhibition de la vidange gastrique induite par l'administration sous-cutanée de CCK 8S dans le protocole décrit dans Life Sciences, 1986, 39, 1631-1638 ; la DE₅₀ (dose efficace 50) ainsi déterminée est nettement inférieure à celle du proglumide, antagoniste connu de la gastrine.

Comme ces composés sont peu toxiques, ils sont utilisables comme médicaments, pour le traitement des désordres physiologiques résultant d'une hypersécrétion de ces peptides ou d'une dysrégulation des systèmes hormonaux biologiques dans lesquels ils sont impliqués, au niveau de la sphère intestinale ou dans le système nerveux central, suivant leur spécificité. On peut se référer à la revue des applications thérapeutiques des antagonistes de CCK et de gastrine publiée dans "Proceedings of International Symposium on Gastrin and Cholecystokinin" 7-11 sept. 1987 - Ed. J.P. Bali, J. Martinez - Elsevier Science Pub. BV.

Notamment les antagonistes de la CCK seront utiles dans le traitement des dyskinésies intestinales, comme le syndrome du colon irritable, dans le traitement des pancréatites aiguës ou chroniques ou dans celui des carcinomes pancréatiques, mais aussi pour régulariser l'appétit, ou, associé aux analgésiques opiacés, dans le traitement de la douleur.

Quant aux antagonistes plus sélectifs de la gastrine, ils seront utiles dans le traitement et la prévention des ulcères gastriques, dans le traitement du syndrome de Zollinger-Ellison, dans celui des hyperplasies des cellules G de l'antre ou pour les malades ayant des tumeurs cancéreuses de l'oesophage, de l'estomac ou de l'intestin.

Parmi les antagonistes de la cholecystokinine au niveau des récepteurs A, on préfère les composés :
2-(1-Carboxyméthyl-2-indolyl)carbonylamino-4-phényl-5-acétoxyéthylthiazole.
- 2-(2-Indolyl)carbonylamino-4-phényl-5-acétoxyéthylthiazole.

Les médicaments selon l'invention comprennent au moins l'un des composés de formule (I) ou l'un de ses sels avec un acide ou une base pharmaceutiquement acceptable, éventuellement associé avec les excipients habituels pour constituer une forme pharmaceutiquement administrable de façon classique par voie orale, transmuqueuse, parentérale ou rectale. Les doses administrées dépendent de la nature et de l'importance de la maladie, du composé et de la voie d'administration. Elles seront généralement comprises entre 20 et 100 mg par jour chez l'homme adulte par voie orale et 3 à 10 mg en injection.

Les compositions pharmaceutiques selon l'invention pourront être, pour l'administration orale, présentées sous forme de comprimés, de pilules, de gélules ou de granulés ou encore de solution, de suspension ou de gel. Pour l'administration parentérale, les compositions de l'invention se présenteront sous forme de solution, de suspension ou d'émulsion dans une huile ou tout solvant injectable, éventuellement à base aqueuse contenant les adjuvants classiques dans ce type de formulation.

Pour l'administration locale, sur la peau ou sur les muqueuses, les compositions selon l'invention se présenteront en crème, pommade ou sous forme de dispositif transdermique, tandis que pour l'administration rectale, elles seront sous forme de suppositoire et de capsule rectale.

Dans ce qui suit, on décrit des exemples de mise en oeuvre de l'invention, ainsi que les procédés de préparation de certains intermédiaires de synthèse de formule Il et IV. Les points de fusion, F, indiqués ont été déterminés en capillaire. Les spectres de résonance magnétique nucléaire, (RMN) ont été enregistrés par rapport au tétraméthylsilane.

### PREPARATION A

### 2-Aminothiazole substitué en position 5 par un groupe -(CH2)mXi·

### 2-Amino-4-(2,4-diméthoxyphényl)-5-benzylthiazole.

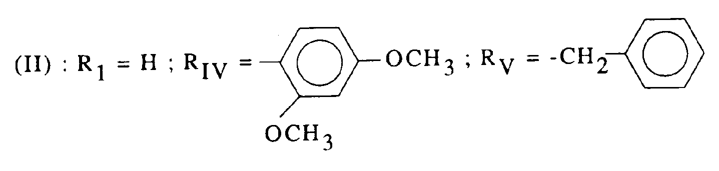

A) La 1-(2,4-diméthoxyphényl)-3-phénylpropan-1-one est préparé selon E. Thomas et al., J. Med. Chem., 1985, 28, 442-446 via la réaction de Friedel-Crafts.
B) La 1-(2,4-diméthoxyphényl)-2-bromo-3-phénylpropan-1-one est préparée selon des méthodes classiques par bromation par le brome dans un solvant tel que le dichlorométhane ou le tétrachlorure de carbone.
C) 2-Amino-4-(2,4-diméthoxyphényl)-5-benzylthiazole.

A 10 g du dérivé bromé préparé précédemment mis en solution dans 100 ml d'éthanol 95°, on ajoute 4,35 g de thiourée et on chauffe le mélange réactionnel à reflux pendant trois heures. On concentre sous vide et reprend le résidu dans le dichlorométhane puis lave avec une solution saturée de Na₂CO₃. La phase organique est séparée par décantation, séchée sur MgS0₄ et concentrée sous vide. Le résidu cristallise dans 50 ml de dichlorométhane.
m=7,10g g
F = 202-203°C.

En procédant comme indiqué ci-dessus, on prépare les 2-aminothiazoles décrits dans le Tableau 1 ci-dessous.

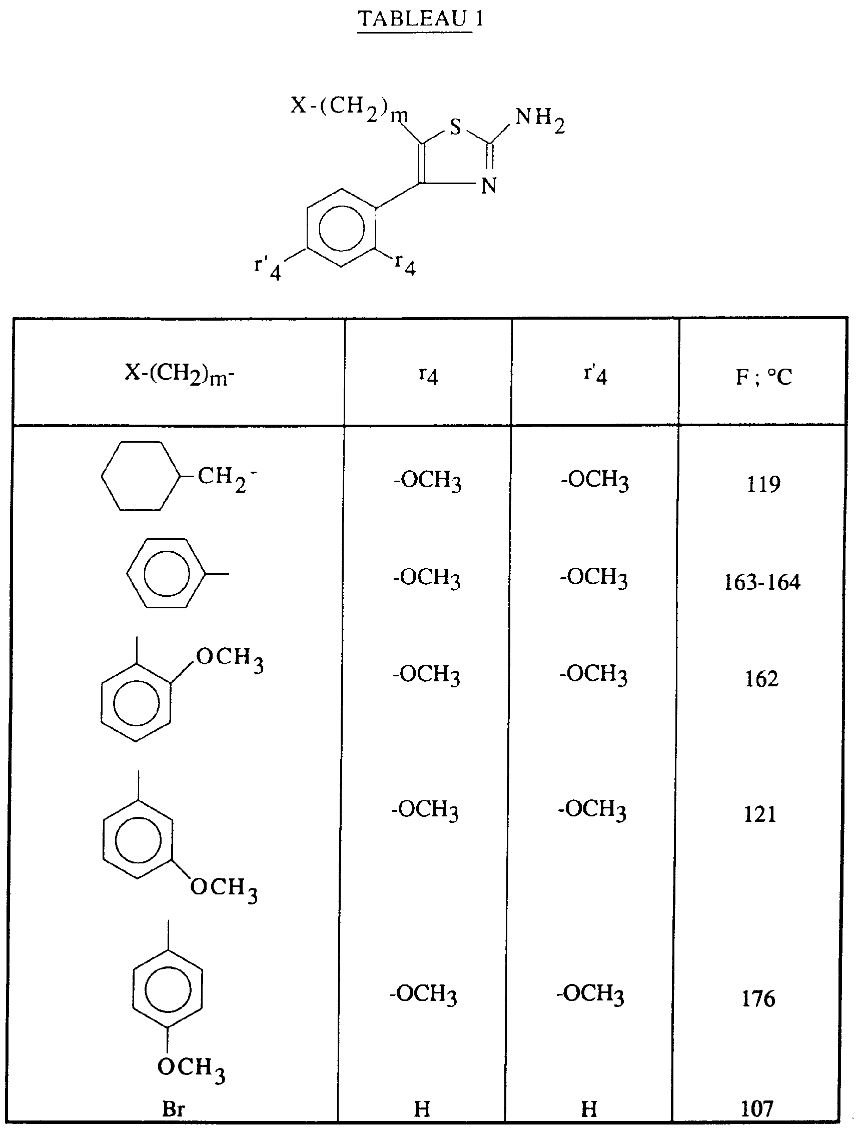

PREPARATION B

### 2-Aminothiazole substitué en position 5 par un groupe -(CH₂)ₘ-CO₂X₁ ou -(CH₂)ₘ-CH₂0H.

### A) 2-Amino-4-phényl-5-méthoxycarbonylméthylthiazole.

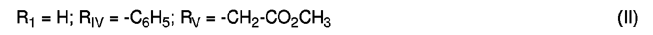

Préparé selon E. Knott, J. Chem. Soc., 1945, 455.

### B) 2-Amino-4-phényl-5-hydroxyéthylthiazole.

A une suspension de 2 g d'hydrure de lithium aluminium dans 100 ml de tétrahydrofurane refroidis à 0°C on ajoute 5 g de l'aminoester préparé précédemment et on chauffe le mélange réactionnel à reflux pendant deux heures. On additionne ensuite après refroidissement au bain de glace successivement 2 ml d'eau, 1 ml de NaOH concentrée et 6 ml d'eau puis agite le mélange réactionnel pendant une nuit. On sépare les minéraux par filtration et concentre les eaux-mères sous vide. Le résidu est repris dans le dichlorométhane, lavé à l'eau et la phase organique est successivement décantée, séchée sur MgS0₄ et concentrée sous vide. Le résidu est chromatographie sur gel de silice, éluant: dichlorométhane/méthanol 100 + 3 (v/v).

La concentration des fractions pures fournit 4 g de l'alcool attendu.

F = 121°C.

En procédant comme indiqué ci-dessus, on prépare les 2-aminothiazoles décrits dans le Tableau 2 ci-dessous.

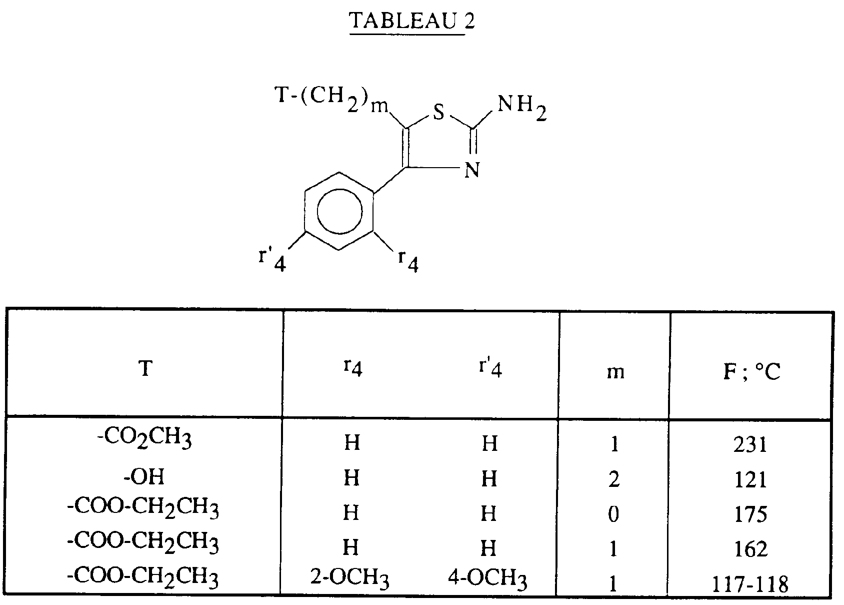

### PREPARATION C

### 2-Aminothiazoles substitués en position 5 par un groupe

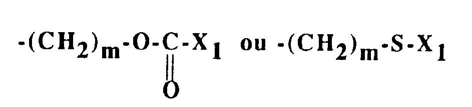

### 2-Amino-5-(1-adamantyl-1-carbonyloxyéthyl)-4-phénylthiazole.

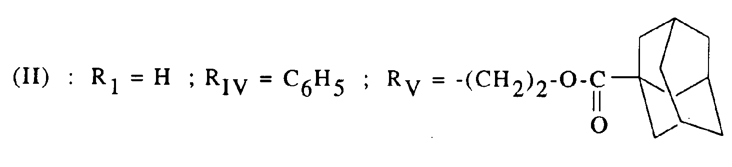

### A. 4-( 1-Adamantylcarbonyloxy)-1-phényl-1-butane.

On prépare selon J. Org. Chem., 1977, 42,8, 1286 le sel de césium de l'acide 1-adamantylcarboxylique à partir de 12 g d'acide 1-adamantylcarboxylique et de 10,96 g de carbonate de césium. Le sel obtenu est mis en solution dans 70 ml de DMF puis on additionne 18 g de 4-iodo-1-phénylbutan-1-one et chauffe le mélange réactionnel à reflux pendant une nuit. On évapore le DMF sous vide, reprend le résidu dans une solution de Na₂C0₃ 5 % et extrait au CH₂CI₂. La phase organique est lavée à l'eau et ensuite séchée sur Na₂S0₄. On concentre sous vide et le résidu est chromatographié sur gel de silice, éluant : CH₂CI₂.

La concentration des fractions pures fournit 10 g du composé attendu.

### B. 2-Amino-5-(1-adamantyl-1-carbonyloxyéthyl)-4-phénylthiazole.

10 g du composé préparé précédemment sont mis en solution dans 100 ml de CCI₄. On ajoute 4,9 g de brome en solution dans 50 ml de CCI₄ et laisse le mélange réactionnel sous agitation pendant 30 minutes. On lave à l'eau, décante la phase organique, la sèche sur MgS0₄, filtre et concentre sous vide. Le résidu est repris dans 50 ml d'étha- nol 95°. On additionne à la solution, 3,9 g de thiourée et laisse le mélange réactionnel pendant une nuit à température ambiante. On concentre le mélange sous vide, reprend le résidu dans le CH₂CI₂, lave avec une solution de NaHC0₃ à 5 %, décante la phase organique, sèche sur MgS0₄, filtre et concentre sous vide. Le résidu est repris dans l'éther et séché.
m = 6,8 g
F = 167°C.

En procédant comme indiqué ci-dessus, on prépare les 2-aminothiazoles décrits dans le Tableau 3 ci-dessous.

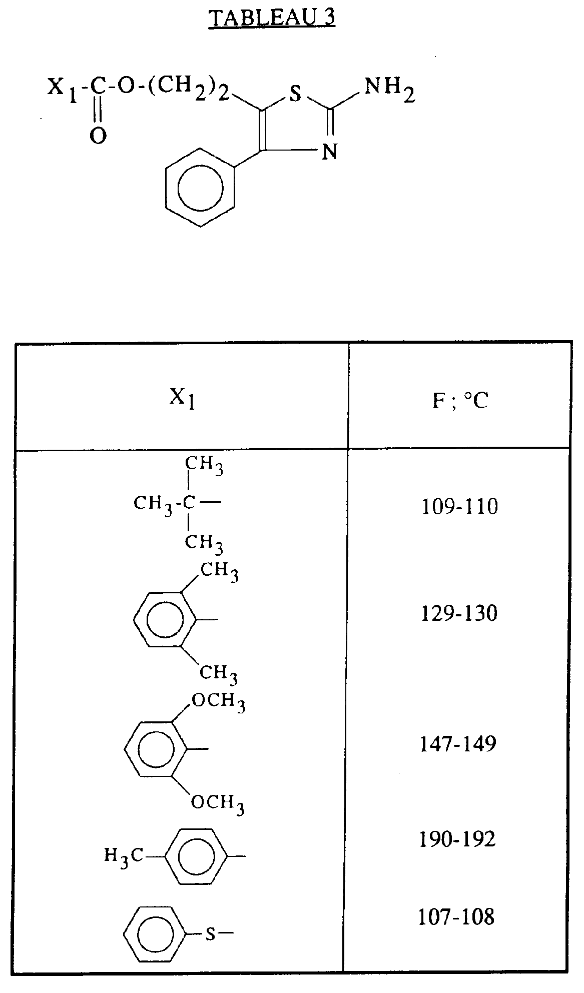

### PREPARATION D

### 2-Aminothiazoles substitués en position 5 par un groupe -(CH₂)ₘX dans lequel X représente un groupe -NX₁X₂ avec X₁ = X₂ = H.

### Préparation du 2-amino-5-aminoéthyl-4-phénylthiazole.

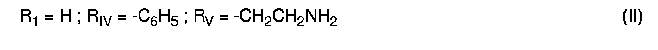

### A) 4-Phtalimido-1-phénylbutan-1-one.

27,4 g de 4-iodo-1-phénylbutan-1-one et 27 g de phtalimide potassique sont chauffés à 120°C pendant 24 heures dans 100 ml de DMF. On concentre le DMF sous vide et le résidu est successivement lavé à l'eau, avec une solution de NaOH 1 N et extrait à l'acétate d'éthyle. Les phases organiques sont décantées, séchées sur MgS0₄, filtrées et concentrées sous vide.
m = 11 g.

### B) 2-Amino-5-phtalimidoéthyl-4-phénylthiazole.

9,6 g du composé préparé précédemment sont mis en solution dans 50 ml de CCl₄ et 80 ml de CH₂CI₂. On additionne goutte à goutte à la solution, une solution de 5,6 g de brome dans 30 ml de CCl₄. Le mélange réactionnel est lavé à l'eau, les phases organiques sont séchées sur MgS0₄, filtrées et concentrées sous vide. Le résidu est repris dans 70 ml d'éthanol, on ajoute 4,5 g de thiourée et laisse le mélange réactionnel à température ambiante pendant une nuit.

Le mélange est refroidi, le bromhydrate est séparé par filtration, lavé à l'éthanol puis agité vigoureusement dans un mélange de Na₂CO₃ à 5 %/éther éthylique. On filtre les cristaux.
m=8g.
F = 208°C

### C) 2-Amino-5-aminoéthyl-4-phénylthiazole.

8 g du produit préparé précédemment sont traités par 1,5 g d'hydrate d'hydrazine en solution dans 100 ml d'éthanol absolu. Le mélange réactionnel est chauffé à reflux pendant une nuit, puis successivement, on concentre l'éthanol sous vide, reprend le résidu dans l'eau, acidifie par addition HCI concentré jusqu'à pH = 1, sépare la phtalazine dione par filtration, alcalinise la phase aqueuse refroidie au bain de glace, par addition de NaOH concentrée jusqu'à pH = 9, filtre le précipité, lave à l'eau et sèche à l'étuve.
m = 3,7 g
F=136-137°C.

### PREPARATION E

### 2-Aminothiazoles substitués en position 5 par un groupe -(CH₂)ₘX dans lequel X représente un groupe - NX₁X₂dans lequel X₁ = H et X₂= -CO-CH₃.

### 2-Amino-5-(2-acétylamino-1-éthyl)-4-phénylthiazole.

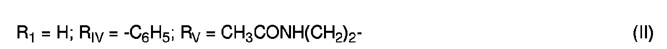

1 g du 2-aminothiazole obtenu selon la préparation D en solution dans 60 ml de THF en présence de 0,7 ml de triéthylamine est traité par 0,44 ml d'anhydride acétique en solution dans 20 ml de THF. Le mélange réactionnel est laissé à température ambiante pendant 2 heures et concentré sous vide. Le résidu est lavé avec une solution de NaHC0₃ 5 %, on sépare le précipité par filtration, lave à l'eau et sèche.
m=₁,₁₂g g
F = 208-209°C.

A partir du 2-aminothiazole obtenu selon la préparation D et en procédant selon la préparation E, on prépare les composés intermédiaires décrits dans le Tableau 4 ci-dessous.

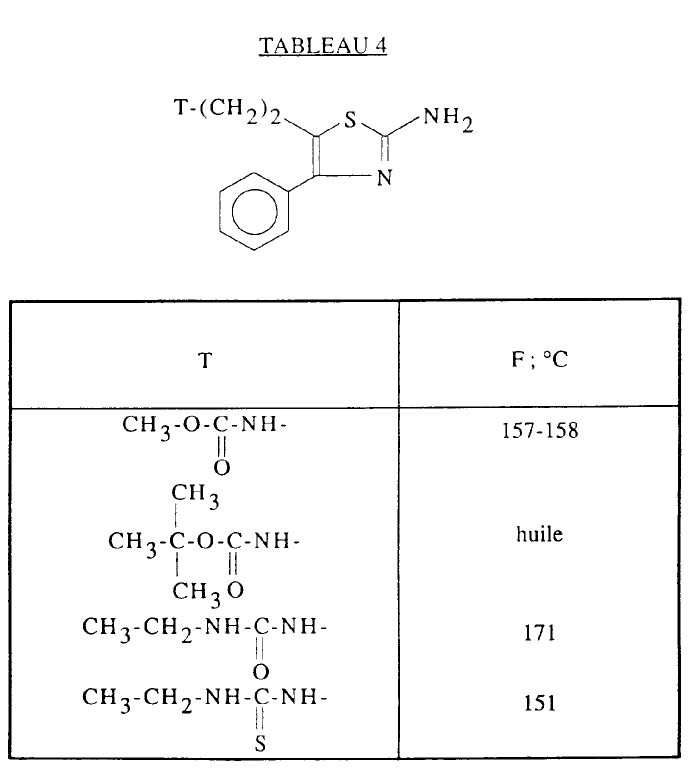

### PREPARATION F

### 2-Amino-4,5-dihydro-[5,4-d]-thiazolo-[1]-benzoxépine.

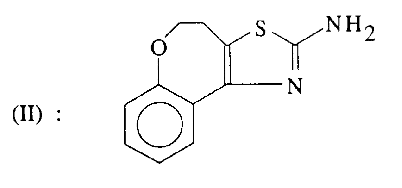

A) La 4-bromo-4-2 H-3,4-dihydro-[1]-benzoxépine-5-one , est préparée selon G. Fontaine, P. Maitte, C.R. Acad. Sci., 1964, 258, 4583.
B) 2-Amino-4,5-dihydro-[5,4-d]-thiazolo [1] benzoxépine.

A 0,027 mole de dérivé bromé solubilisé dans 100 ml d'éthanol, on ajoute 2,05 g de thiourée. Le mélange est chauffé à reflux pendant 3 heures. L'éthanol est évaporé, le résidu est repris par une solution aqueuse de carbonate de sodium. On extrait à l'acétate d'éthyle, sèche la phase organique sur Na₂S0₄ et évapore à sec. On obtient 2,4 g de cristaux blancs.

F = 216°C.

### PREPARATION G

### 2-Aminothiazoles substitués en position 5 par un groupe -(CH₂)ₘ-X dans lequel m = 0 et X représente un groupe alcoxy en C₁-C_{5,} un halogène.

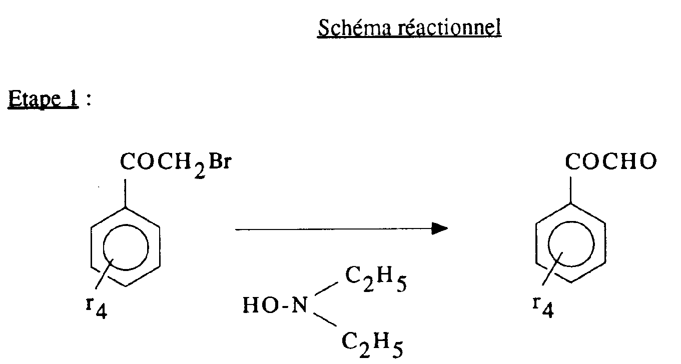

selon J. Org. Chem., 1977,42 (4), 754. 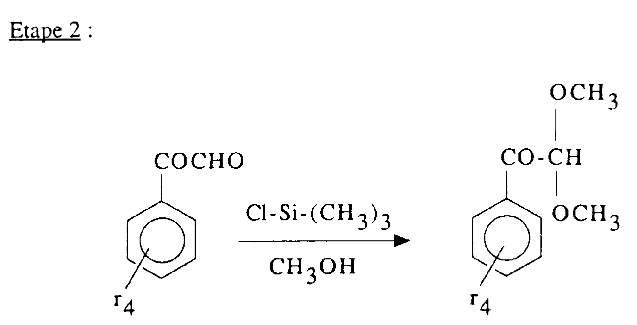 selon Synthesis, 1983, 203. 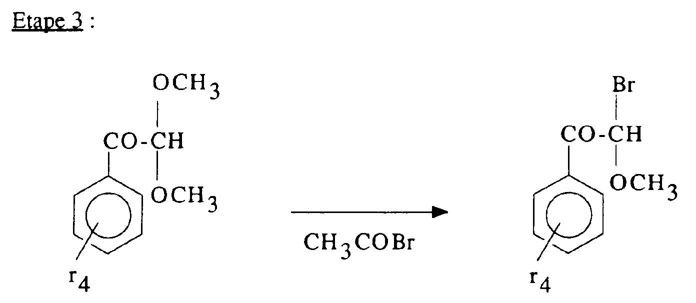 selon J. Chem. Soc., Perkin I, 1981, 2435. 2-Amino-5-méthoxy-4-phénylthiazole.

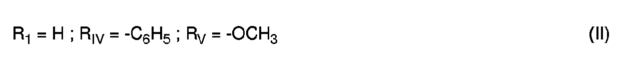

15,65 g de 2-bromo-2-méthoxy-1-phényléthanone et 5,52 g de thiourée sont mis en solution dans 70 ml de méthanol. Le mélange réactionnel est chauffé à reflux pendant une nuit puis concentré sous vide. Le résidu est repris dans une solution de Na₂CO₃ à 10 % dans l'eau, on extrait au CH₂CI₂, sépare la phase organique qui est successivement séchée sur Na₂SO₄, filtrée et concentrée sous vide. Le résidu est recristailisé de l'éther isopropylique.
m = ₇,₅ g
F = 96°C.

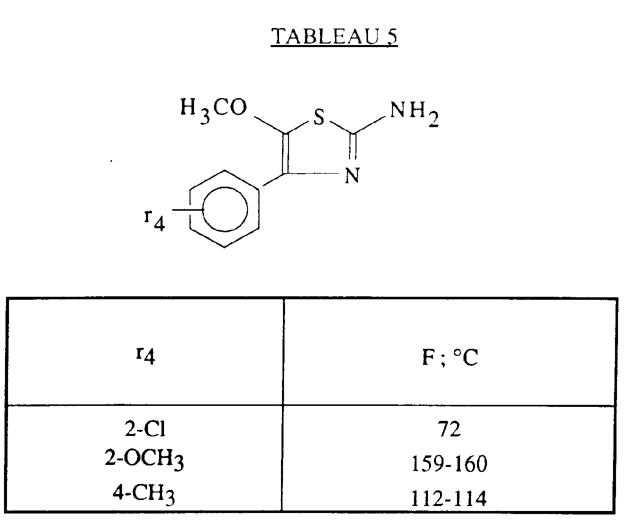

### PREPARATION H

Préparation des acides indolecarboxyliques :
A) Acide 1-tert-butyloxycarbonylméthylindole-2-carboxylique.
a) Indole-2-carboxylate de benzyle.

On introduit 5 g de N,N'-carbonyldiimidazole dans une solution de 5 g d'acide indole-2-carboxylique dans 50 ml de tétrahydrofurane sec ; après 12 heures d'agitation à température ambiante, on ajoute 3,7 g d'alcool benzylique et on chauffe le mélange réactionnel à sa température de reflux ; celle-ci est maintenue durant 8 heures, avant d'éliminer le solvant par distillation sous pression réduite. Le résidu est dissous dans l'acétate d'éthyle et la phase organique lavée par une solution aqueuse de NaOH 1 N puis séchée avant évaporation du solvant.

Le résidu jaune est recristallisé dans l'isopropanol.
F 136°C
R = 85 %.

### b) 1-tert-butoxycarbonylméthylindole-2-carboxylate de benzyle.

A une solution de (0,072 mode ; 18,18 g) d'indole-2-carboxylate de benzyle dans 200 ml de diméthylformamide, sous atmosphère d'azote, on ajoute par portions (0,075 mode ; 2,25 g) d'hydrure de sodium à 80 % dans l'huile, à une température comprise entre 0°C et 5°C. On laisse revenir le mélange à température ambiante et on agite le mélange pendant 1 heure. On additionne ensuite goutte à goutte, à 10°C, (0,072 mole ; 14 g de bromoacétate de tert-butyle. Le mélange réactionnel est laissé pendant 3 heures à température ambiante. On évapore le diméthylformamide, reprend successivement à l'eau, extrait au chlorure de méthylène, sèche la phase organique sur sulfate de sodium et évapore à sec. Par cristallisation du résidu dans l'éther diisopropylique on obtient 23,8 g de cristaux blancs.

F = 95°C.

### c) Acide 1-tert-butoxycarbonylméthylindole-2-carboxylique.

L'ester préparé précédemment (0,065 mole ; 23,8 g) est solubilisé dans un mélange de 400 ml d'éthanol et 100 ml de diméthylformamide. On ajoute 1 g de palladium sur charbon à 5 % et hydrogène sous pression atmosphérique à température ambiante. Après 30 minutes d'agitation le volume théorique d'hydrogène est absorbé. On filtre sur talc le catalyseur, évapore à sec les solvants. On obtient un résidu cristallisé que l'on lave par l'éther diisopropylique. On obtient 15,3 g de cristaux blancs.

F = 177°C.

### B) Acide 1-acétylindole-2-carboxylique.

Un mélange de (0,06 mole; 10 g) d'acide indole-2-carboxylique, (0,15 mole; 21,25 g) de triéthylamine, (0,075 mole ; 7,5 g) d'anhydride acétique et (0,006 mole ; 0,8 g) de 4-diméthylaminopyridine dans le chlorure de méthylène est agité à température ambiante pendant 18 heures. Le mélange réactionnel est ensuite versé sur une solution aqueuse de tampon pH = 2. Le précipité formé, est filtré puis séché à l'étuve sous vide. La phase chlorure de méthylène est décantée, séchée sur sulfate de sodium et évaporée aux 3/4. Un deuxième jet d'acide 1-acétylindole-2-carboxylique précipite. Les deux jets sont réunis pour fournir 9,4 g de cristaux beiges.

F = 168°C.

### C) Acide 1-benzyloxycarbonylaminoindole-2-carboxylique.

On dissout 8 g d'acide indole-2-carboxylique dans 120 ml de dichlorométhane puis on ajoute 10 g de triéthylamine et 1 g de 4-diméthylaminopyridine.

Le mélange réactionnel est agité puis refroidi à 0°-5°C. On ajoute goutte à goutte 8,5 g de chlorure de benzyloxy- carbonyle à une température inférieure à 5°C. Le mélange est laissé sous agitation pendant une nuit puis concentré sous vide. Le résidu est repris dans 500 ml d'acétate d'éthyle puis filtré. Les eaux-mères sont concentrées sous vide et reprises dans 50 ml de dichlorométhane. On filtre et concentre les eaux-mères sous vide.
m = 2,4 g d'huile
RMN (DMSO): 2 H à 5,38 (s, CH₂-C₆H₅); 10 H entre 7,0 et 8,0 (m; Har).

### D) Acide 1-tert-butyloxycarbonylindole-2-carboxylique.

On introduit goutte à goutte 30 ml d'une solution de 6 g de dicarbonate de ditertiobutyle dans 30 ml d'une solution de 4 g d'acide indole-2-carboxylique, 4 ml de triéthylamine et 0,4 g de 4-diméthylaminopyridine dans l'acétonitrile. Après 2 heures d'agitation à température ambiante et élimination du précipité formé, l'acétonitrile est éliminé par distillation et le résidu est dissous dans le chlorure de méthylène. La phase organique est lavée à l'eau, séchée, et concentrée à sec.
F = 117°C ; rendement 66 %.
R = 66 %.

### EXEMPLE 1

### 2-(Indolyl-2-carbonylamino)-4-(2,4-diméthoxyphényl)-5-benzylthiazole.

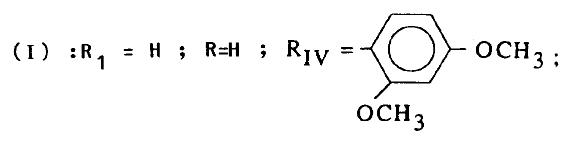

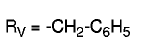

1,96 g de 2-amino-4-(2,4-diméthoxyphényl)-5-benzylthiazole préparé précédemment selon la préparation A, 1,22 g d'acide 1-acétylindole-2-carboxylique, 0,85 g de triéthylamine et 2,95 g de BOP sont mis en solution dans 20 ml de diméthylformamide.

Le mélange réactionnel est agité pendant 24 heures à température ambiante puis versé sur une solution tampon pH = 2. Un précipité jaune est séparé par filtration, lavé à l'eau et mis en solution dans l'acétate d'éthyle. La solution est lavée successivement avec une solution tampon pH = 2, à l'eau, avec une solution de NaHC0₃ 5 % et à l'eau, puis séchée sur MgS0₄ et concentrée sous vide. Le résidu est purifié par chromatographie sur gel de silice, éluant : dichlorométhane/acétate d'éthyle 98/2 (v/v).

La concentration des fractions de produit pur fournit un résidu qui est traité sous agitation pendant 24 heures avec 3 g de Na₂CO₃ dans 80 ml de méthanol. Le méthanol est concentré sous vide et le résidu est repris dans un mélange eau/éther. Un précipité blanc est séparé par filtration et lavé à l'éther.
m = ₀,₉₇ g
F =201-202°C.

De la même façon on prépare les composés selon l'invention, décrits dans le tableau 6 ci-après.

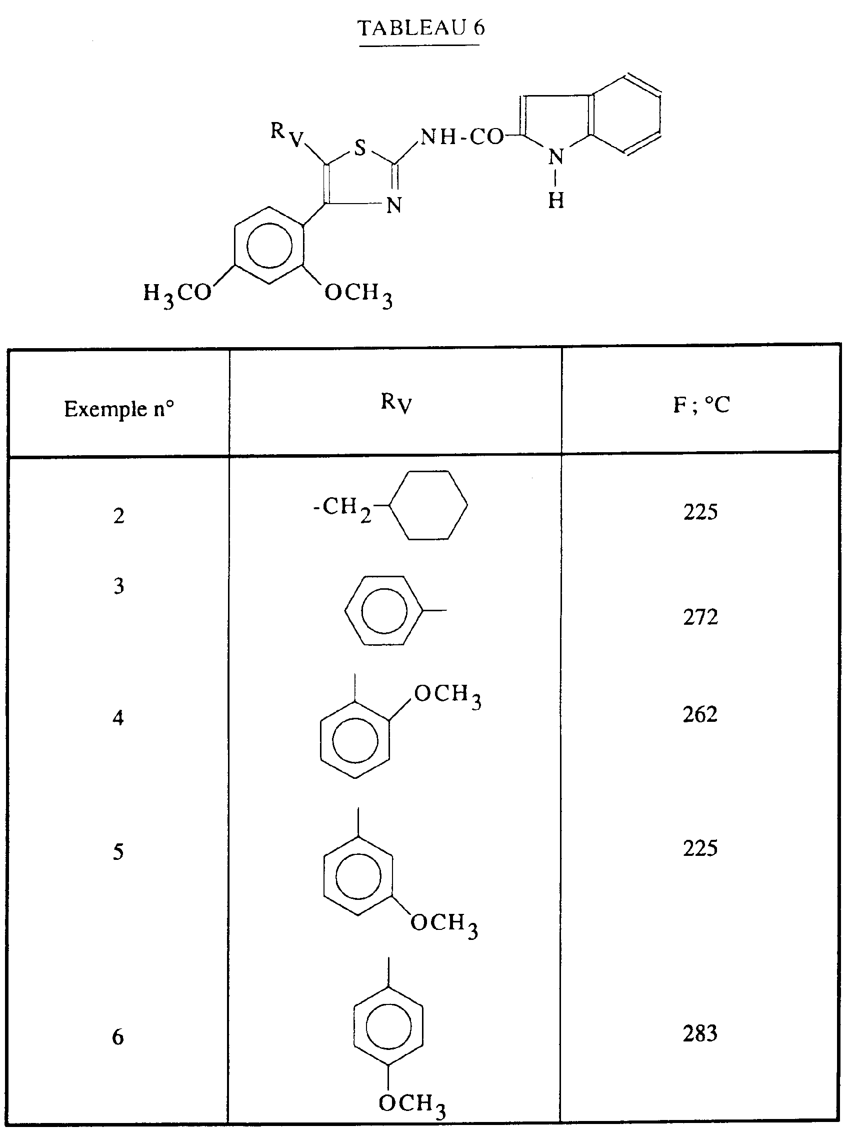

### EXEMPLE 7

### 2-(1-tert-butoxycarbonyl - méthyl-2-indolyl)carbonylamino-4-phényl-5-hydroxyéthylthiazole.

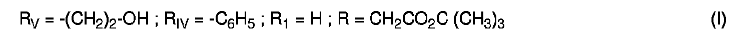

2 g de l'aminoalcool préparé précédemment (selon la préparation B, Tableau 2), 2,75 g d'acide 1-tert-butoxycarbo- nylméthylindole-2-carboxylique, 1,4 g de triéthylamine et 4,9 g de BOP sont mis en solution dans 15 ml de diméthylformamide. Après une nuit à température ambiante, le mélange réactionnel est versé sur du tampon phosphate pH = 2. Un précipité est séparé par filtration, lavé à l'eau et dissout dans l'acétate d'éthyle. La solution est successivement lavée avec une solution de NaHCO₃ à 5 % à l'eau, séparée par décantation, la phase organique est séchée sur MgS0₄ et concentrée sous vide.

Le résidu est purifié par chromatographie sur gel de silice, éluant : dichlorométhane/méthanol 100 + 0,5 (v/v).

Le produit élué en premier correspond au composé diacylé (0 et N acylation, F = 70°C).

Le produit attendu est élué en second.
m = 1,2 g
F 180-181°C.

### EXEMPLE 8

### 2-(1-tert-butoxycarbonylméthyl-2-indolyl)carbonylamino-4-phényl-5-acétoxyéthylthiazole.

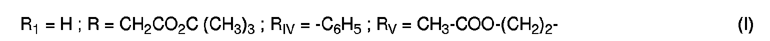

0,30 g du produit préparé précédemment sont mis en suspension dans 5 ml de pyridine.

On additionne 1.2 ml d'anhydride acétique et agite le mélange réactionnel pendant une nuit à température ambiante. Le mélange est alors versé sur du tampon sulfate pH = 2 et un précipité est séparé par filtration, lavé à l'eau puis repris dans le dichlorométhane. La solution est successivement lavée avec une solution de NaHCO₃ à 5 %, séparée par décantation, la phase organique est séchée sur MgS0₄, filtrée et concentrée sous vide. Le résidu est purifié par chromatographie sur gel de silice, éluant: dichlorométhane/acétate d'éthyle 98/2 (v/v).
m = 0,16 g
RMN (DMSO) : 9 H à 1,48 (S, t-B_{U}0₂C) ; 3 H à 2,00 (S, CH₃C0₂) ; 2 H à 3,24 (T, J = 7 Hz, CH₂ thiazole) ; 2 H à 4,30 (T, J = 7 Hz, CH₂0Ac) ; 2 H à 5,40 (S, CH₂CO₂ t-Bu) ; 10 H entre 7,2 et 7,9 (M, Har) ; 1 H à 12,8 (S, NHCO).

### EXEMPLE 9

### 2-(1-Carboxylméthyl-2-indolyl)carbonylamino-4-phényl-5-acétoxyéthylthiazole.

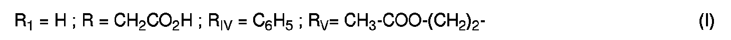

0,15 g du composé préparé précédemment sont solubilisés dans 2 ml d'anisole et 10 ml d'acide trifluoroacétique.

Le mélange est laissé pendant 45 minutes à température ambiante puis concentré sous vide. Le résidu obtenu est lavé par un mélange d'hexane et de diéthyléther (50/50) puis séché.
m = 0,14 g
F = 217-218°C.

### EXEMPLE 10

### 2-(1-Acétyl-2-indolyl)carbonylamino-4-(2,4-diméthoxyphényl)-5-éthoxycarbonylméthylthiazole.

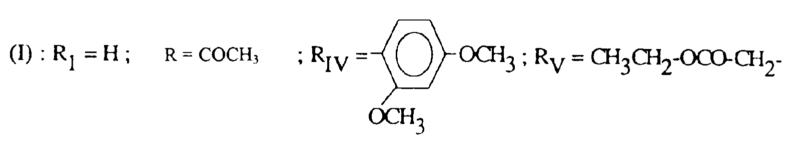

1,5 g de 2-amino-4-(2,4-diméthoxyphényl)-5-éthoxycarbonylméthylthiazole, 1 g d'acide 1-acétylindole-2-carboxylique, 0,7 ml de triéthylamine et 2,39 g de BOP sont mis en solution dans 15 ml de dichlorométhane. Le mélange réactionnel est agité pendant une nuit à température ambiante puis concentré sous vide. Le résidu est repris dans l'acétate d'éthyle et la solution est successivement lavée avec une solution tampon pH = 2, avec une solution de NaHCO₃ à 5 % et à l'eau, puis la phase organique est séchée sur MgS0₄ et concentrée sous vide. Le résidu est purifié par chromatographie sur gel de silice, éluant: dichlorométhane/acétate d'éthyle 100/2,5 (v/v).
m = 1,2 g
F =130-135°C.

### EXEMPLE 11

### 2-(2-Indolyl)carbonylamino-5-éthoxycarbonyl-4-phénylthiazole.

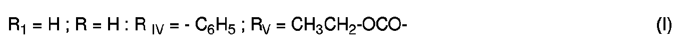

On prépare, en procédant selon l'exemple 10 précédemment décrit le composé 2-(1-acétyl-2-indolyl)carbonylamino-4-phényl-5-éthoxycarbonylthiazole (1,5 g) qui est dissout dans 100 ml d'éthanol en présence de 0,6 g de Na₂CO₃. Le mélange est agité à température ambiante pendant 48 heures puis concentré sous vide. Le résidu est trituré dans l'eau puis dans le minimum de dichlorométhane, filtré et séché.
m = 1,1 g
F = 248°C.

### EXEMPLE 12

### 2-(2-Indolyl)carbonylamino-4-(2,4-diméthoxyphényl)-5 carboxyméthylthiazole.

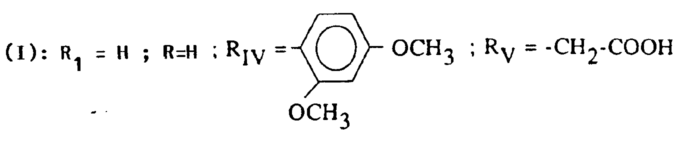

0,5 g de 2-(1-acétyl-2-indolyl)carbonylamino-4-(2,4-diméthoxyphényl)-5-éthoxycarbonylméthylthiazole préparé précédemment selon l'exemple 10 sont mis en solution dans 10 ml d'éthanol 95° puis on additionne 1,5 ml de NaOH 2N. Le mélange réactionnel est agité pendant une nuit à température ambiante puis concentré sous vide. Le résidu est repris dans une solution tampon pH = 2, un précipité est séparé par filtration , lavé à l'eau, filtré, puis rincé à l'éther.
m = 0,28 g
F = 284°C.

En procédant selon les exemples 7 à 12 ci-dessus, on prépare les composés décrits dans le Tableau 7 ci-dessous.

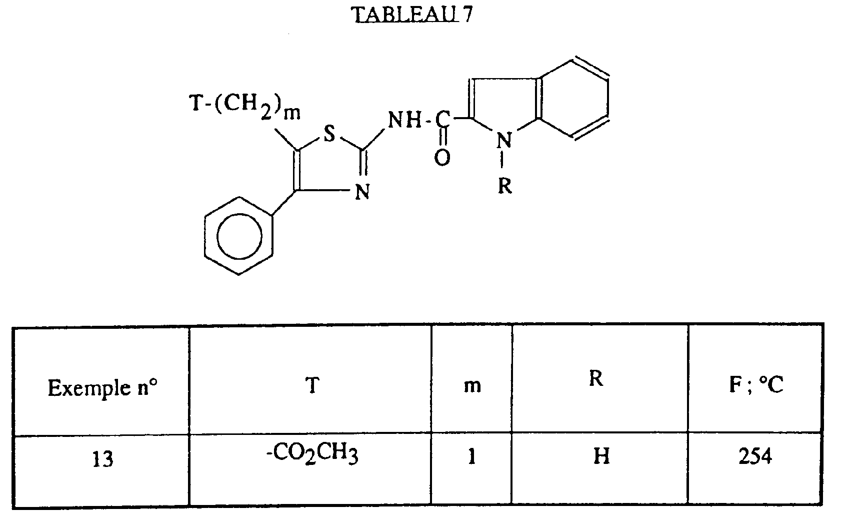

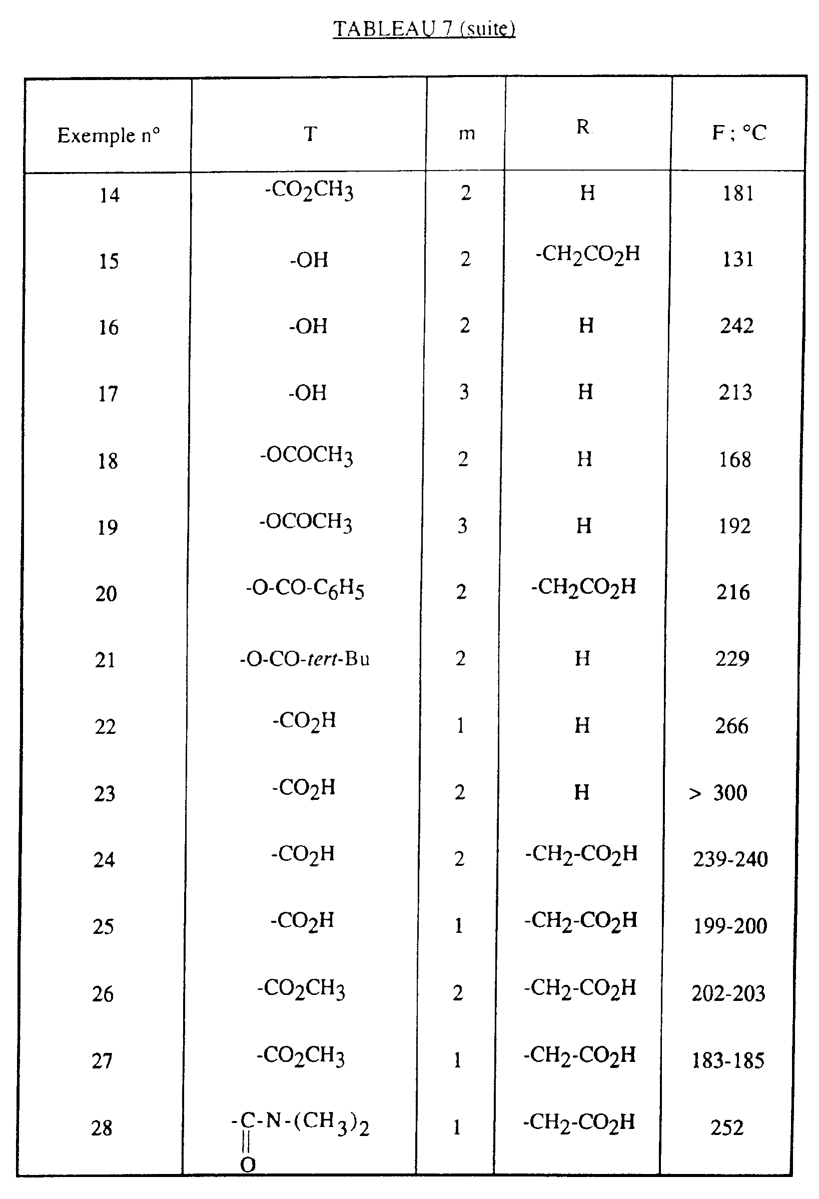

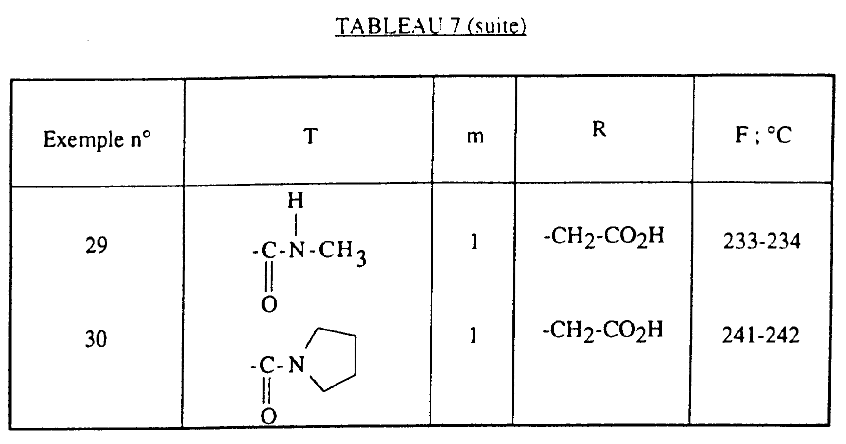

### EXEMPLE 31

### 2-(2-Indolyl)carbonylamino-4-phényl-5-[2-(1-pyrrolidinocarbonyl)-1-éthyl]thiazole.

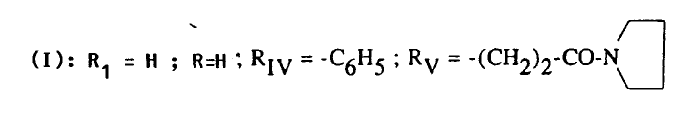

0,5 g de l'ester décrit à l'exemple 14 est ajouté dans 5 ml de pyrrolidine, le mélange est agité pendant une nuit à température ambiante, puis versé dans une solution tampon pH = 2. Un précipité est séparé par filtration puis dissout dans l'acétate d'éthyle. La solution est lavée avec une solution tampon pH = 2 puis à l'eau, la phase organique est séparée par décantation séchée sur MgS0₄ et concentrée sous vide.
m = ₀,48 g
F = 179_{°}C.

En procédant selon l'exemple 10 ci-dessus, on prépare les composés 32 à 51 décrits dans le Tableau 8 ci-dessous.

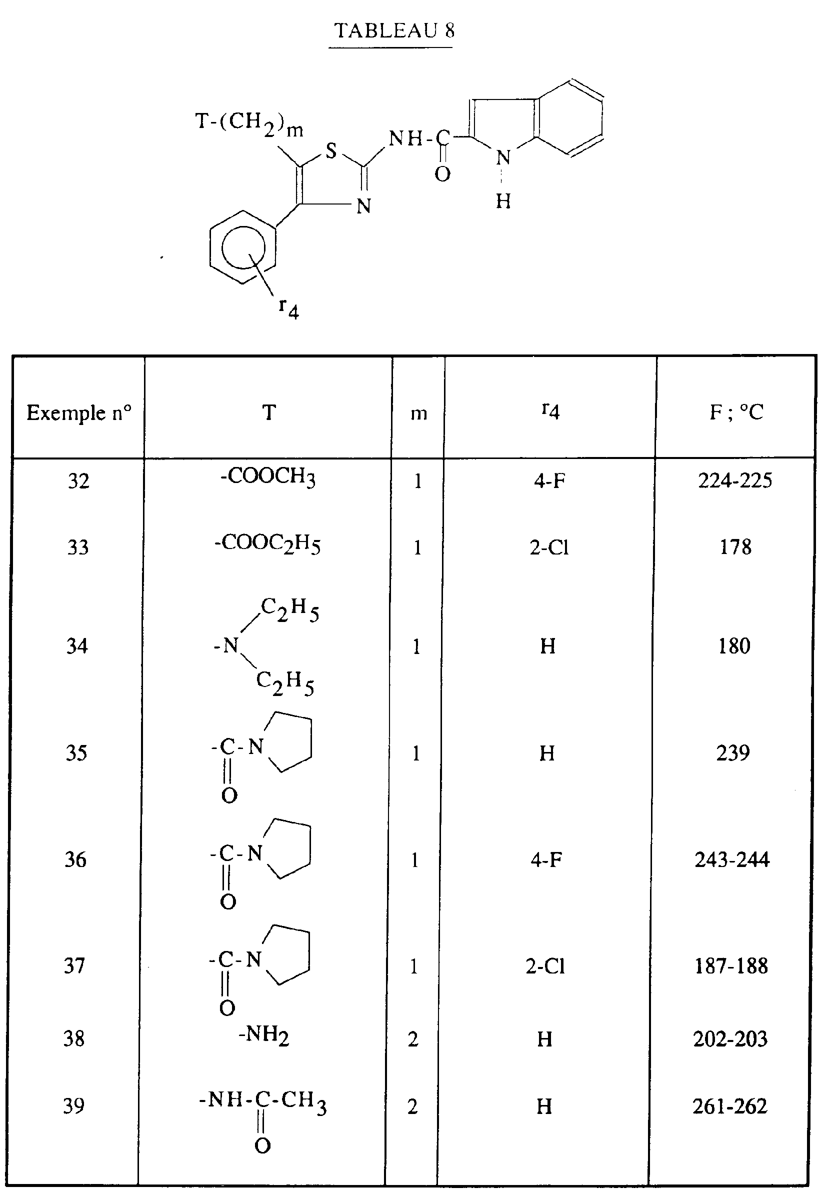

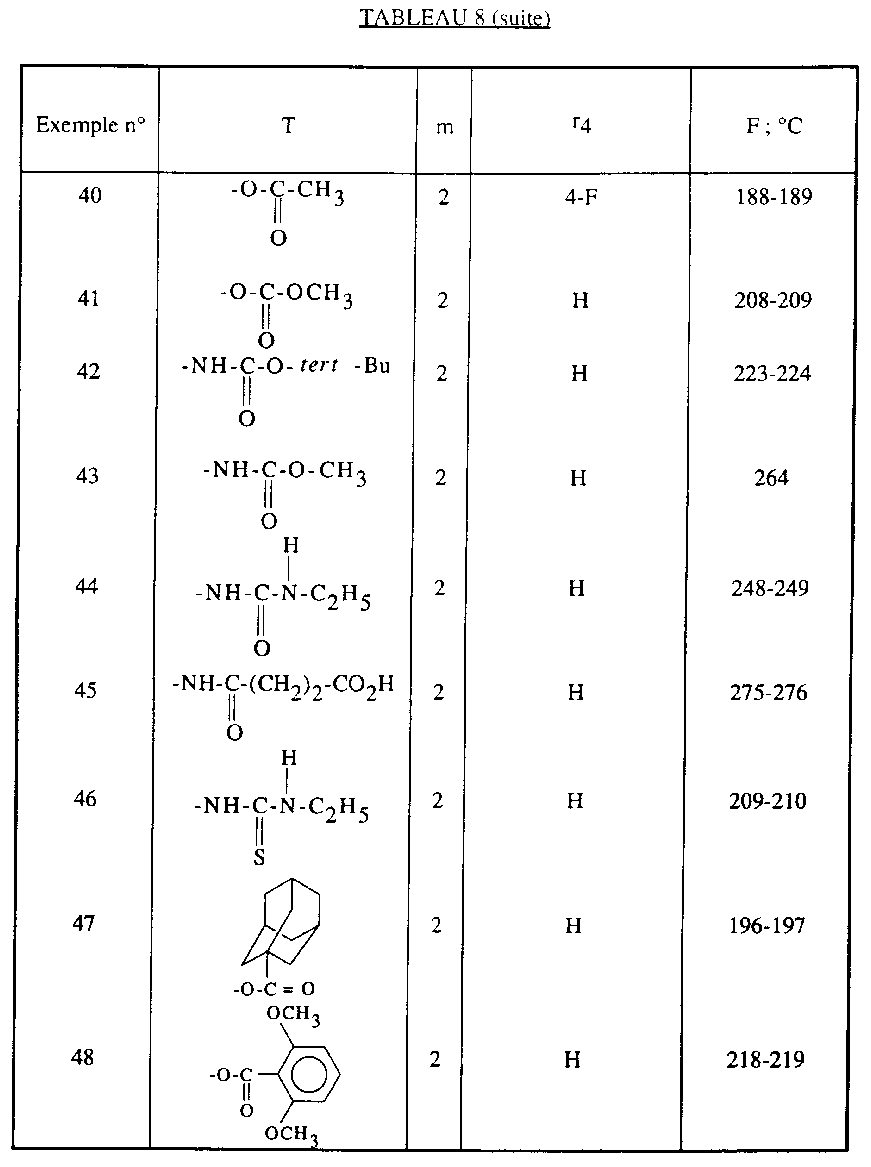

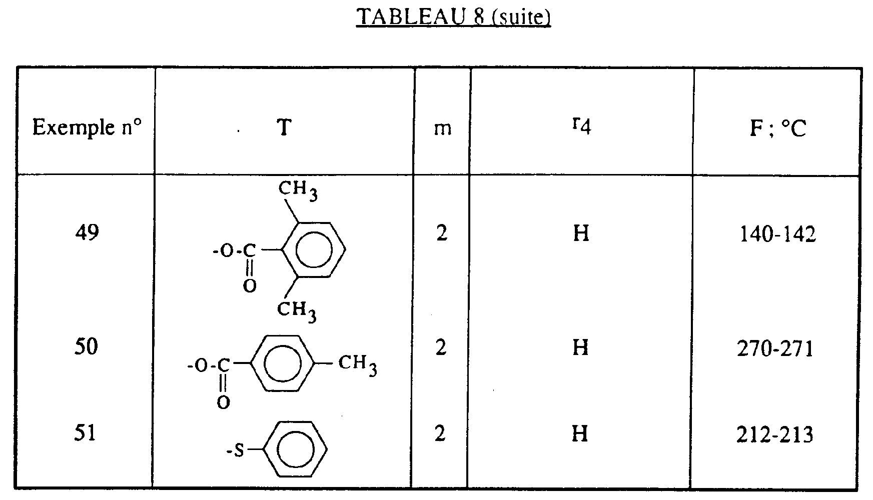

### EXEMPLE 52

### 2-(2-Indolyl)carbonylamino-4-phényl-5-phénylaminocarbonyloxyéthylthiazole.

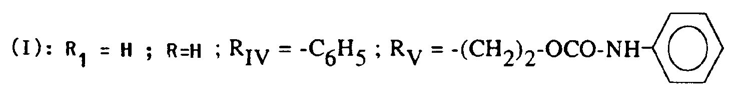

### A) 2-(1-Acétyl-2-indolyl)carbonylamino-4-phényl-5-phénylaminocarbonyloxyéthylthiazole.

0,7 g de l'alcool préparé selon l'exemple 16 et 0,2 ml d'isocyanate de phényle sont agités pendant une nuit à température ambiante dans 5 ml de dichlorométhane. Le précipité formé est séparé par filtration puis purifié par chromatographie sur gel de silice, éluant : dichlorométhane/acétate d'éthyle 95/5 (v/v).
m = ₀,₅₂ g
F =156°C.

### B) Composé 52

0,5 g du composé préparé précédemment sont mis en solution dans 30 ml d'éthanol et 5 ml d'eau puis on ajoute 0,21 g de Na₂CO₃.

Le mélange réactionnel est agité pendant une nuit à température ambiante puis concentré sous vide. Le résidu est repris dans l'acétate d'éthyle et on lave successivement avec une solution de Na₂CO₃ et à l'eau. La phase organique est séparée par décantation et concentrée sous vide. Le résidu est repris dans l'éther.
m = ₀,₄ g
F = _{249°}C.

### EXEMPLE 53

### 2-(2-Indolyl)carbonylamino-4,5-dihydro-[5,4-d]- 1 Hthiazolobenzoxépine.

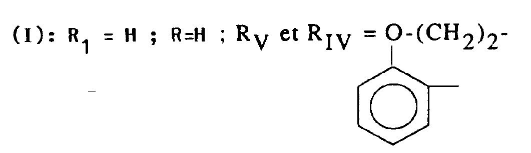

Dans 30 ml de diméthylformamide on mélange 1 g du thiazole préparé précédemment (Préparation F), 2,6 g de BOP, 0,93 g d'acide 1-acétylindole-2-carboxylique et 0,46 g de triéthylamine. Le mélange réactionnel est agité 30 heures à température ambiante. On évapore le diméthylformamide, reprend le résidu dans l'acétate d'éthyle et lave à l'eau. La phase organique est séchée sur sulfate de sodium et évaporée. Le résidu est chromatographié sur gel de silice, éluant : dichlorométhanelméthanol 100 + 0,5 (v/v). On obtient 0,9 g d'une mousse jaune que l'on solubilise dans du dichlorométhane additionné d'éthanol (100 ml). On ajoute 10 ml de NaOH 2N et agite le mélange à température ambiante pendant 1 heure. Après évaporation des solvants organiques, on reprend le résidu par l'acétate d'éthyle et lave avec une solution tampon pH = 2. On sèche la phase organique sur MgS0₄, filtre et évapore. On obtient des cristaux jaunes qui sont ensuite lavés par le dichlorométhane puis l'éthanol.
m = ₀,45 g
F> 260°C.

### EXEMPLE 54

### 2-(2-Indolyl)carbonylamino-4-phényl-5-( 1-pipéridinyl)thiazole.

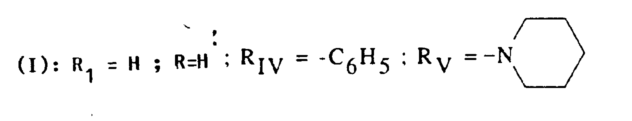

### A) 2-Amino-4-phényl-5-(1-pipéridinyl)thiazole.

On chauffe à reflux pendant 48 heures un mélange de 1 g de 2-amino-4-phényl-5-bromothiazole et de 1,7 g de pipéridine dans 25 ml d'éthanol absolu. L'éthanol est concentré sous vide et le résidu est repris dans 50 ml d'eau et 10 ml de NaOH à 30 %. On extrait à l'acétate d'éthyle, sèche la phase organique sur Na₂C0₄ et filtre. On concentre sous vide et recristallise le résidu de l'éther isopropylique.
m = ₀,₄₁ g
F =135-137°C.

### B) Composé 54

0,4 g du produit obtenu précédemment sont mis en solution dans 50 ml de dichlorométhane. On ajoute successivement 0,33 g d'acide 1-acétylindole-2-carboxylique, 0,82 g de BOP et 0,19 g de triéthylamine. Le mélange réactionnel est agité pendant 4 jours à température ambiante. On ajoute 25 ml d'eau et sépare la phase organique par décantation, on sèche sur Na₂S0₄ et concentre sous vide. Le résidu est repris dans 50 ml d'éthanol absolu, on ajoute 10 ml de NaOH 2,5N et agite le mélange pendant 3 heures à température ambiante. On concentre sous vide, reprend le résidu dans 50 ml d'eau, on extrait à l'acétate d'éthyle, sépare la phase organique par décantation, sèche sur Na₂S0₄ et concentre sous vide. Le résidu est recristallisé de l'acétate d'éthyle.
m = ₀,₂₆ g
F > 260°C.

En procédant selon l'exemple 54 on prépare les composés 55 et 56 décrits dans le Tableau 9 ci-dessous.

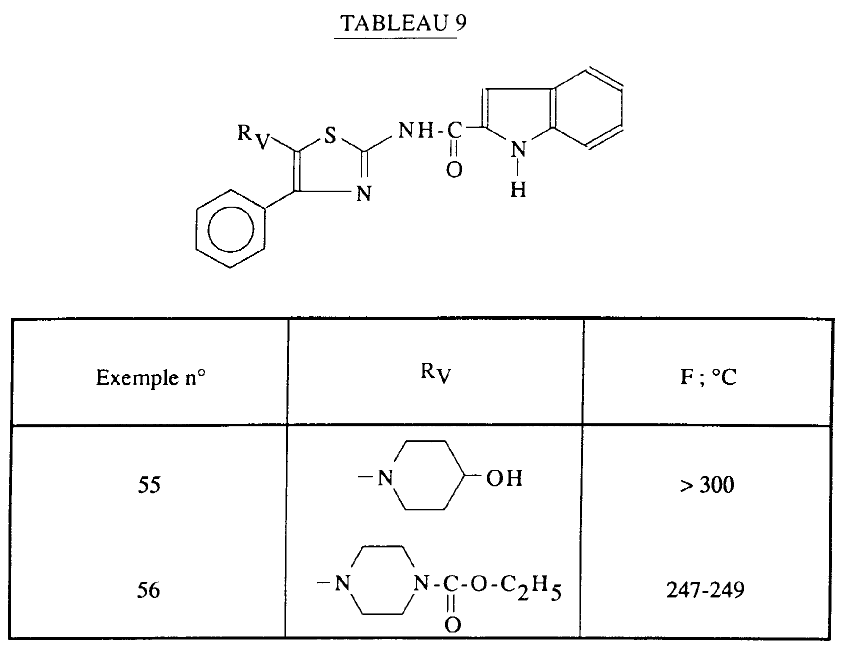

### EXEMPLE 57

### 2-(2-Indolyl)carbonylamino-5-bromo-4-phénylthiazole.

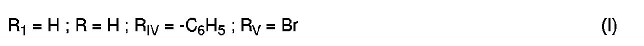

On dissout 3,9 g (0,015 mole) de 2-amino-5-bromothiazole dans 60 ml de dichlorométhane.

On ajoute 3,26 g d'acide 1-acétylindole-2-carboxylique, 8,1 g de BOP, 1,85 g de triéthylamine, on agite le mélange réactionnel à température ambiante pendant 8 jours puis on ajoute 100 ml d'une solution aqueuse tamponnée à pH = 2, on décante, sèche la phase organique sur Na₂S0₄ et concentre sous vide. On reprend le résidu dans 100 ml de méthanol puis on additionne 5 g de Na₂CO₃. On agite pendant trois heures à température ambiante, concentre sous vide a froid, reprend par 100 ml d'une solution aqueuse tamponnée à pH = 2, extrait à l'acétate d'éthyle et sèche, sur Na₂S0₄ la phase organique. On filtre et concentre sous vide. On chromatographie le résidu sur gel de silice, éluant : CH₂CI₂. Après élimination d'impuretés de tête, on élue le produit attendu que l'on recristallise après évaporation du solvant, d'un mélange CH₂CI₂/éther diisopropylique.
m = 2,8 g
F = 224-226°C.

### EXEMPLE 58

### 2-(Indolyl-2)carbonylamino-5-méthoxy-4-phénylthiazole.

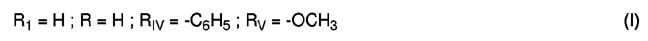

3,15 g de 2-amino-5-méthoxy-4-phénylthiazole sont dissous dans 60 ml de CH₂CI₂. On ajoute 3,26 g d'acide 1-acétylindole-2-carboxylique, 8,12 g de BOP et 1,86 g de triéthylamine et on agite le mélange réactionnel à température ambiante pendant une semaine. On ajoute 50 ml d'eau, décante la phase organique, sèche sur Na₂S0₄, filtre et concentre sous vide. Le résidu est repris dans 100 ml de méthanol, on ajoute 10 g de K₂CO₃ et agite le mélange pendant une nuit à température ambiante. On concentre sous vide, reprend le résidu dans l'eau, sépare le précipité formé par filtration et le lave au CH₂CI₂.
m = 1,7 g
F > 260°C.

En procédant selon l'exemple ci-dessus, on prépare les composés 59 à 61 décrits dans le Tableau 10 ci-dessous.

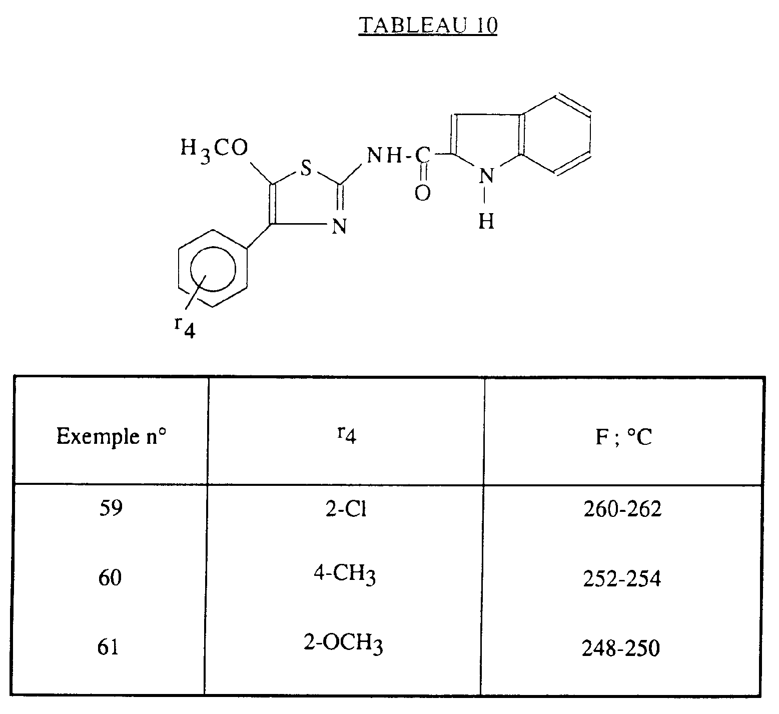

### EXEMPLE 62

### 2-(Indolyl-2)carbonylamino-5-hydroxy-4-phénylthiazole.

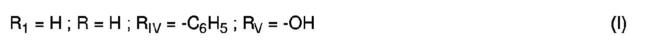

0,58 g du thiazole préparé selon l'exemple 58 sont mis en solution dans 100 ml de CH₂CI₂. On ajoute à température ambiante 4,98 ml d'une solution 2M de tribromure de bore dans le CH₂CI₂ et on laisse le mélange réactionnel sous agitation pendant 24 heures. On ajoute à nouveau 4,98 ml de BBr₃, laisse le mélange pendant 5 jours à température ambiante et ajoute une dernière fois 4,98 g de BBr₃ en abandonnant le mélange réactionnel pendant 48 heures à température ambiante. Ce dernier est alors amené à pH = 5-6 par addition d'une solution de NaOH 4N puis on extrait la phase organique avec 2 fois 50 ml de NaOH 4N. On neutralise la phase aqueuse en additionnant une solution d'HCI 2N. On extrait au CH₂CI₂, sèche les phases organiques sur Na₂SO₄, filtre et concentre sous vide pour obtenir un résidu qui cristallise.
m=0,5 g
F = 237-239°C.

## Revendications (Revendications pour l'(les) Etat(s) contractant(s) suivant(s) : AT, BE, CH, DE, DK, FR, GB, IT, LI, LU, NL, PT, SE)

1. Acylamino-2 thiazoles de formule : 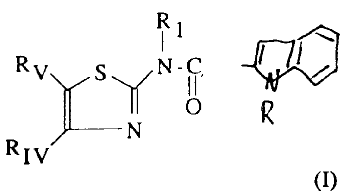 dans laquelle
- R₁ représente l'atome d'hydrogène ; un groupe alkyle en C₁ à C₄ ou un groupe aminoalkylène de formule -Z₁-NR₄R₅ dans laquelle Z₁ représente un alkylène en C₂ à C₄ et R₄ et R₅ représentent indépendamment H ou un alkyle en Ci à C₄ ;
- R_{IV} représente un groupe phényle non substitué ou portant un ou plusieurs substituants choisis parmi les atomes d'halogène, les groupes (C₁-C₆) alkyle, et alcoxy en Ci à C_{3,} ou R_{IV} et R_{V} considérés ensemble représentent le groupe : 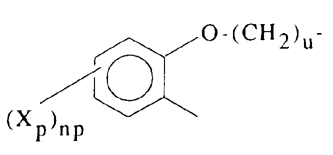 fixé par le carbone du phényle en position 4 du noyau thiazolyle et dans lequel u vaut 1 à 3, portant éventuellement un ou plusieurs (np) substituants Xp, identiques ou différents choisis parmi les atomes d'halogène, les groupes alkyle et alcoxy en Ci à C₃, les groupes nitro et trifluorométhyle, np valant de 0 à 3 ;
- R_{V} représente un groupe -(CH₂)ₘ-X dans lequel m est 0 à 5 et X représente
un atome d'halogène, un hydroxyle, un cycloalkyle en C₃ à C₇, un phényle qui peut être substitué par un des groupes choisis parmi les atomes d'halogène, les groupes alkyles ou alcoxy en C₁ à C_{3,} nitro, amino, hydroxy ou trifluorométhyle ;
un groupe choisi parmi -COOH ; -COOX₁ ; -O-COX₁ ; -SCOX₁ ; (O)_{q}-S-X₁ avec q = 0,1 ou 2 ; -O-COOX₁ ; 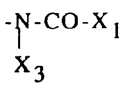 avec X₃ = H ou alkyle en C₁ à C₃ ; 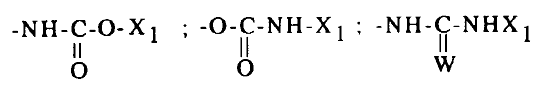 avecW=OouS ; 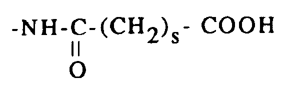 avec s = 2,3,4
dans lequel X₁ représente un alkyle en C₁ à C₅ ; un phényle qui peut être substitué par un ou des groupes choisis parmi les atomes d'halogène, les groupes alkyles ou alcoxy en C₁ à C₃, nitro, amino, hydroxy, trifluorométhyle ; un groupe adamantyle ;
un groupe choisi parmi -CONX₁X₂ ; -NX₁X₂ ; dans lequel X₁ représente l'hydrogène, un alkyle en Ci à C₃ ou un phényle non substitué ou substitué par un ou des groupes choisis parmi les atomes d'halogène, les groupes alkyles ou alcoxy en C₁ à C₃, nitro, amino, hydroxy, trifluorométhyle et X₂ représente un atome d'hydrogène, un alkyle en C₁ à C_{3,} ou bien, X₁ et X₂ constituent avec l'atome d'azote auquel ils sont liés un hétérocycle choisi parmi la pyrrolidine ou la pipéridine non substitué ou substitué par un groupe oxo ou par un groupe hydroxyle, ce dernier étant non substitué ou substitué par un acyle, par un groupe -COOX₁ ou -CONX₁X₂ ;
ou encore R_{V} représente un alcoxy en C₁-C₅ ; un groupe hydroxyle ; une amine cyclique à 5 ou 6 chaînons non substituée ou substituée par un groupe oxo ou un groupe hydroxyle ; un groupe pipérazinyle non substitué ou N-substitué par un groupe -COOAlk dans lequel Alk représente un alkyle en C₁-C₅ ; un groupe acide carboxylique, un groupe -NX₂X₄ avec X₄ = H ou X₄ = -(CH₂)ₜ-X_{5,} avec t égal à 2, 3 ou 4 et X₅ représente -OH, -O-CO-R₂, -NHCOR₂, 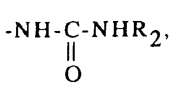
dans lequel R₂ représente un alkyle C₁-C₆ ; ou un groupe -NR₂R₃ avec R₂ ou R₃ représentant indépendamment H, alkyle en Ci à C₆, un groupe phényle non substitué ou substitué par un ou plusieurs substituants choisis parmi les atomes d'halogène, un groupe alkyle en C₁ à C₃, un groupe alcoxy en Ci à C₃ ou R₂ et R₃ constituent avec l'atome d'azote auquel ils sont liés un hétérocycle à 5 ou 6 chainons ;
- R représente H, (C₁-C₄) alkyle, carboxyalkylène -Z₄-COOR₁₀ dans laquelle Z₄ représente (C₁-C₄) alkylène et R₁₀ est H, benzyle ou (Ci-C₆) alkyle ; carbamoyalkylène -Z₅-CONR₁₁ R₁₂ dans laquelle Z₅ représente (C₁-C₄) alkylène et R₁₁ et R₁₂ représentent indépendamment H, (C₁-C₆) alkyle, ou forment avec N un hétérocycle saturé comme morpholino ou pipéridino; acyle COR₁₃ avec R₁₃ représente (C₁-C₄) alkyle ou phényle ; alcoxycarbonyle -COOR₁₄ avec R₁₄ étant tert-butyle ou benzyle;
ainsi que les sels d'addition des composés de formule (I) avec des acides et des bases minérales ou organiques.

2. Composés selon la revendication 1 de formule (I) dans laquelle lorsque R_{IV} représente un groupe phényle éventuellement substitué, le ou les atomes d'halogène sont choisis parmi le fluor et le chore.

3. Composés selon la revendication 1 de formule (I) dans laquelle R_{IV} représente un phényle.

4. Procédé pour la préparation des 2-acylamino-5-thiazoles substitues selon la revendication 1, caractérisé en ce que l'on traite un 2-amino-5-thiazole substitué de formule : 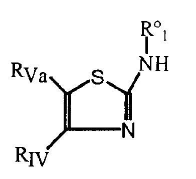 dans laquelle R°₁ représente les mêmes substituants que R₁ dans lequel le groupe amino présent est N-protégé, Rᵥₐ représente les mêmes substituants que R_{V} dans lesquels les groupes hydroxy ou amino sont 0- et N-protégés, avec un dérivé fonctionnel de l'acide de formule : 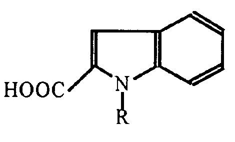 dans lequel le groupe NH est N-protégé, puis,
- lorsque R°₁ ou Rᵥₐ contiennent des groupes N-protégés ou O-protégés, on déprotège et on soumet éventuellement le produit ainsi obtenu à une acylation ou alkylation pour obtenir les composés selon l'invention pour lesquels les substituants R₁, Rivet Rᵥ sont tels que définis pour (1), ou un de leurs sels.

5. Procédé de préparation des composés selon l'une des revendications 1 à 3, de formule (I) dans laquelle R est H, caractérisé en ce que l'on prépare le composé correspondant de formule I dans laquelle R représente H par acylation de l'aminothiazole avec une forme activée de l'acide de formule : 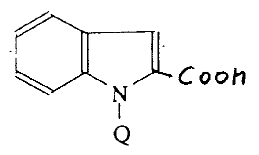 dans laquelle Q représente un groupe protecteur, pour obtenir le dérivé indolyle correspondant, après avoir déprotégé l'azote.

6. Composé de formule : 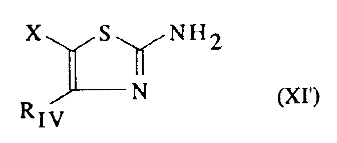 dans lequel R_{IV} est tel que défini dans la revendication 1 et X représente un alcoxy en C₁-C_{5,} une pipéridine non substituée ou substituée par un 4-hydroxy ou une 4-alcoxycarbonylpipérazine dans laquelle l'alcoxy est en C₁-C₃.

7. Composé de formule : 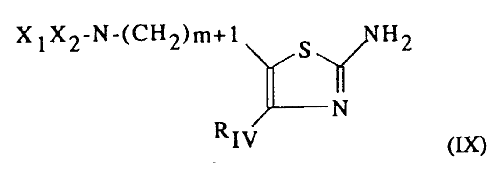 dans laquelle R_{IV} est tel que défini dans la revendication 1, m est 1 ou 2 et X₁X₂-N représente un groupe phtalimido ou -NH₂ ou un de ses sels.

8. Composition pharmaceutique, caractérisée en ce qu'elle comprend au moins un composé selon l'une des revendications 1 à 3 associé à au moins un excipient.

9. Composés selon la revendication 1 de formule (I) dans laquelle Rᵥ représente -(CH₂)ₘ-X où X est un atome de brome.

## Revendications (Revendications pour l'(les) Etat(s) contractant(s) suivant(s) : ES)

1. Procédé de préparation d'acylamino-2 thiazoles de formule : 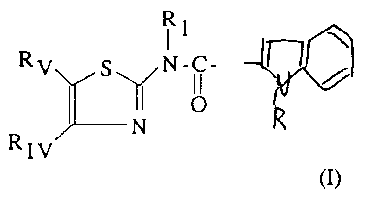 dans laquelle
- R₁ représente l'atome d'hydrogène ; un groupe alkyle en C₁ à C₄ ; un groupe aminoalkylène de formule -Z₁-NR₄R₅ dans laquelle Z₁ représente un alkylène en C₂ à C₄ et R₄ et R₅ représentent indépendamment H ou un alkyle en Ci à C₄
- R_{IV} représente un groupe phényle non substitué ou portant un ou plusieurs substituants choisis parmi les atomes d'halogène, les groupes (C₁-C₆) alkyle, et alcoxy en Ci à C₃, ou R_{IV} et R_{V} considérés ensemble représentent le groupe : 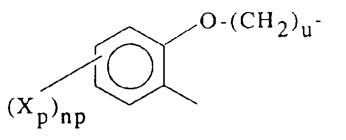 fixé par le carbone du phényle en position 4 du noyau thiazolyle et dans lequel u vaut 1 à 3, portant éventuellement un ou plusieurs (np) substituants Xp, identiques ou différents choisis parmi les atomes d'halogène, les groupes alkyle et alcoxy en C₁ à C₃, les groupes nitro et trifluorométhyle, np valant de 0 à 3 ;
- Rᵥ représente un groupe -(CH₂)ₘ-X dans lequel m est 0 à 5 et X représente
un atome d'halogène, un hydroxyle, un cycloalkyle en C₃ à C₇, un phényle qui peut être substitué par un des groupes choisis parmi les atomes d'halogène, les groupes alkyles ou alcoxy en Ci à C₃, nitro, amino, hydroxy ou trifluorométhyle ;
un groupe choisi parmi -COOH ; -COOX₁ ; -0-COX₁ ; -SCOX₁ ; (O)_{q}-S-X₁ avec q = 0,1 ou 2 ; -O-COOX₁ ; 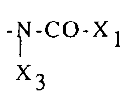 avec X₃ = H ou alkyle en C₁ à C₃ ; 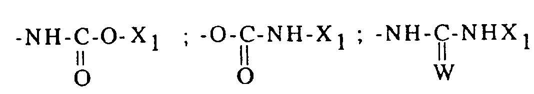 avec W = O ou S ; 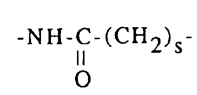 COOH avec s = 2,3,4 dans lequel X₁ représente un alkyle en C₁ à C₅ ; un phényle qui peut être substitué par un ou des groupes choisis parmi les atomes d'halogène, les groupes alkyles ou alcoxy en C₁ à C₃, nitro, amino, hydroxy, trifluorométhyle ; un groupe adamantyle ;
un groupe choisi parmi -CONX₁X₂ ; -NX₁X₂ ; dans lequel X₁ représente l'hydrogène, un alkyle en C₁ à C₃ ou un phényle non substitué ou substitué par un ou des groupes choisis parmi les atomes d'halogène, les groupes alkyles ou alcoxy en C₁ à C₃, nitro, amino, hydroxy, trifluorométhyle et X₂ représente un atome d'hydrogène, un alkyle en C₁ à C₃, ou bien, X₁ et X₂ constituent avec l'atome d'azote auquel ils sont liés un hétérocycle choisi parmi la pyrrolidine ou la pipéridine non substitué ou substitué par un groupe oxo ou par un groupe hydroxyle, ce dernier étant non substitué ou substitué par un acyle, par un groupe -COOX₁ ou -CONX₁X₂ ;
ou encore Rᵥ représente un alcoxy en C₁-C₅ ; un groupe hydroxyle ; une amine cyclique à 5 ou 6 chaînons non substituée ou substituée par un groupe oxo ou un groupe hydroxyle ; un groupe pipérazinyle non substitué ou N-substitué par un groupe -COOAlk dans lequel Alk représente un alkyle en C₁-C₅ ; un groupe acide carboxylique, un groupe -NX₂X₄ avec X₄ = H ou X₄ = -(CH₂)ₜ X_{5,} avec t égal à 2, 3 ou 4 et X₅ représente -OH, -O-CO-R₂, -NHCOR₂, 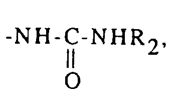
dans lequel R₂ représente un alkyle C₁-C₆ ; ou un groupe -NR₂R₃ avec R₂ ou R₃ représentant indépendamment H, alkyle en Ci à C₆, un groupe phényle non substitué ou substitué par un ou plusieurs substituants choisis parmi les atomes d'halogène, un groupe alkyle en C₁ à C₃, un groupe alcoxy en C₁ à C₃ ou R₂ et R₃ constituent avec l'atome d'azote auquel ils sont liés un hétérocycle à 5 ou 6 chainons ;
- R représent H, un (C₁-C₄) alkyle, carboxyalkylène -Z₄-COOR₁₀ dans laquelle Z₄ représente (C₁-C₄) alkylène et R₁₀ est H, benzyle ou (C₁-C₆) alkyle ; carbamoyalkylène -Z₅-CONR₁₁R₁₂ dans laquelle Z₅ représente (C₁-C₄) alkylène et R₁₁ et R₁₂ représentent indépendamment H, (Ci-C₆) alkyle, ou forment avec N un hétérocycle saturé comme morpholino ou pipéridino ; acyle COR₁₃ avec R₁₃ représente (C₁-C₄) alkyle ou phényle ; alcoxycarbonyle -COOR₁₄ avec R₁₄ étant tert-butyle ou benzyle;
ainsi que les sels d'addition des composés de formule (I) avec des acides et des bases minérales ou organiques,
caractérisé en ce que l'on traite un 2-amino-5-thiazole substitué de formule 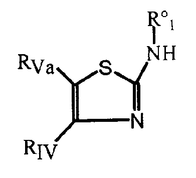
dans laquelle R°₁ représente les mêmes substituants que R₁ dans lequel le groupe amino présent est N-protégé, Rᵥₐ représente les mêmes substituants que Rᵥ dans lesquels les groupes hydroxy ou amino sont 0- et N-protégés, avec un dérivé fonctionnel de l'acide de formule : 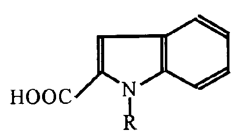
dans lequel le groupe NH est N-protégé, puis
lorsque R°₁, Rᵥₐ contiennent des groupes N-protégés ou O-protégés, on déprotège et on soumet éventuellement le produit ainsi obtenu à une acylation ou alkylation pour obtenir les composés selon l'invention pour lesquels les substituants R₁, R_{IV} et R_{V} sont tels que définis pour (1), ou un de leurs sels.

2. Procédé selon la revendication 1, caractérisé en ce que dans la formule (I) lorsque R_{IV} représente phényle éventuellement substitué, le ou les atomes d'halogène sont choisis parmi le fluor et le chlore.

3. Procédé selon la revendication 1, caractérisé en ce que dans la formule (1) R_{IV} représente un phényle.

4. Procédé de préparation des composés selon la revendication 3, de formule (I) dans laquelle Z représente le noyau indole, non substitué sur l'azote et éventuellement substitué sur le phényle, caractérisé en ce que l'on prépare le composé correspondant de formule I dans laquelle R représente H par acylation de l'aminothiazole avec une forme activée de l'acide de formule : 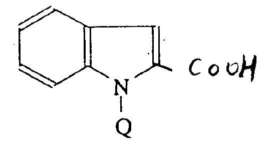 dans laquelle Q représente un groupe protecteur, obtenir le dérivé indolyle correspondant, après avoir déprotégé l'azote.

## Revendications (Revendications pour l'(les) Etat(s) contractant(s) suivant(s) : GR)

1. Acylamino-2 thiazoles de formule : 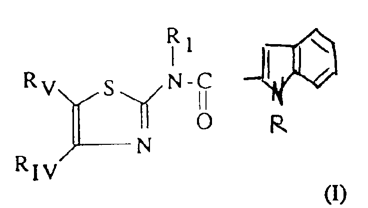 dans laquelle
- R₁ représente l'atome d'hydrogène ; un groupe alkyle en C₁ à C₄ ou un groupe aminoalkylène de formule -Z₁-NR₄R₅ dans laquelle Z₁ représente un alkylène en C₂ à C₄ et R₄ et R₅ représentent indépendamment H ou un alkyle en C₁ à C₄ ;
- R_{IV} représente un groupe phényle non substitué ou portant un ou plusieurs substituants choisis parmi les atomes d'halogène, les groupes (C₁-C₆) alkyle, et alcoxy en C₁ à C₃, ou R_{IV} et R_{V} considérés ensemble représentent le groupe : 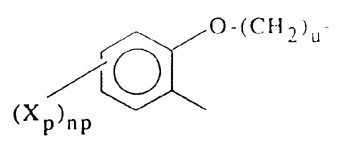 fixé par le carbone du phényle en position 4 du noyau thiazolyle et dans lequel u vaut 1 à 3, portant éventuellement un ou plusieurs (np) substituants Xp, identiques ou différents choisis parmi les atomes d'halogène, les groupes alkyle et alcoxy en C₁ à C₃, les groupes nitro et trifluorométhyle, np valant de 0 à 3 ;
- Rᵥ représente un groupe -(CH₂)ₘ-X dans lequel m est 0 à 5 et X représente
un atome d'halogène, un hydroxyle, un cycloalkyle en C₃ à C₇, un phényle qui peut être substitué par un des groupes choisis parmi les atomes d'halogène, les groupes alkyles ou alcoxy en C₁ à C₃, nitro, amino, hydroxy ou trifluorométhyle ; un groupe choisi parmi -COOH ; -COOX₁ ; -0-COX₁ ; -SCOX₁ ; (O)_{q}-S-X₁ avec q = 0,1 ou 2 ; -O-COOX₁ ; 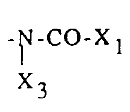 avec X₃ = H ou alkyle en C₁ à C3 ; 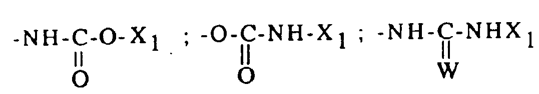 avecW=OouS ; 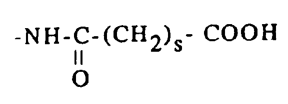 avec s = 2,3,4
dans lequel X₁ représente un alkyle en C₁ à C₅ ; un phényle qui peut être substitué par un ou des groupes choisis parmi les atomes d'halogène, les groupes alkyles ou alcoxy en C₁ à C₃, nitro, amino, hydroxy, trifluorométhyle ; un groupe adamantyle ;
un groupe choisi parmi -CONX₁X₂ ; -NX₁X₂ ; dans lequel X₁ représente l'hydrogène, un alkyle en C₁ à C₃ ou un phényle non substitué ou substitué par un ou des groupes choisis parmi les atomes d'halogène, les groupes alkyles ou alcoxy en C₁ à C₃, nitro, amino, hydroxy, trifluorométhyle et X₂ représente un atome d'hydrogène, un alkyle en Ci à C₃, ou bien, X₁ et X₂ constituent avec l'atome d'azote auquel ils sont liés un hétérocycle choisi parmi la pyrrolidine ou la pipéridine non substitué ou substitué par un groupe oxo ou par un groupe hydroxyle, ce dernier étant non substitué ou substitué par un acyle, par un groupe -COOX₁ ou -CONX₁X₂ ;
ou encore Rᵥ représente un alcoxy en C₁-C₅ ; un groupe hydroxyle ; une amine cyclique à 5 ou 6 chaînons non substituée ou substituée par un groupe oxo ou un groupe hydroxyle ; un groupe pipérazinyle non substitué ou N-substitué par un groupe -COOAlk dans lequel Alk représente un alkyle en C₁-C₅ ; un groupe acide carboxylique, un groupe -NX₂X₄ avec X₄ = H ou X₄ = -(CH₂)ₜ-X₅, avec t égal à 2, 3 ou 4 et X₅ représente -OH, -O-CO-R₂, -NHCOR₂, 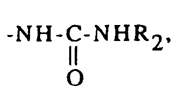
dans lequel R₂ représente un alkyle Ci-C₆ ; ou un groupe -NR₂R₃ avec R₂ ou R₃ représentant indépendamment H, alkyle en Ci à C₆, un groupe phényle non substitué ou substitué par un ou plusieurs substituants choisis parmi les atomes d'halogène, un groupe alkyle en C₁ à C₃, un groupe alcoxy en Ci à C₃ ou R₂ et R₃ constituent avec l'atome d'azote auquel ils sont liés un hétérocycle à 5 ou 6 chainons ;
- R représente H, (C₁-C₄) alkyle, carboxyalkylène -Z₄-COOR₁₀ dans laquelle Z₄ représente (C₁-C₄) alkylène et R₁₀ est H, benzyle ou (Ci-C₆) alkyle ; carbamoyalkylène -Z₅-CONR₁₁ R₁₂ dans laquelle Z₅ représente (C₁-C₄) alkylène et R₁₁ et R₁₂ représentent indépendamment H, (Ci-C₆) alkyle, ou forment avec N un hétérocycle saturé comme morpholino ou pipéridino; acyle COR₁₃ avec R₁₃ représente (C₁-C₄) alkyle ou phényle ; alcoxycarbonyle -COOR₁₄ avec R₁₄ étant tert-butyle ou benzyle;
ainsi que les sels d'addition des composés de formule (I) avec des acides et des bases minérales ou organiques.

2. Composés selon la revendication 1 de formule (I) dans laquelle lorsque R_{IV} représente un groupe phényle éventuellement substitué, le ou les atomes d'halogène sont choisis parmi le fluor et le chore.

3. Composés selon la revendication 1 de formule (1) dans laquelle R_{IV} représente un phényle.

4. Procédé pour la préparation des 2-acylamino-5-thiazoles substitués selon la revendication 1, caractérisé en ce que l'on traite un 2-amino-5-thiazole substitué de formule : 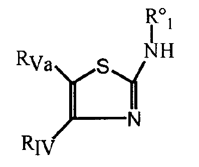 dans laquelle R°₁ représente les mêmes substituants que R₁ dans lequel le groupe amino présent est N-protégé, Rᵥₐ représente les mêmes substituants que Rᵥ dans lesquels les groupes hydroxy ou amino sont O- et N-protégés, avec un dérivé fonctionnel de l'acide de formule : 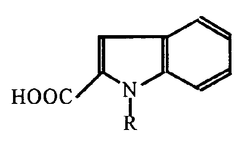 dans lequel le groupe NH est N-protégé, puis
- lorsque R°₁ ou Rᵥₐ contiennent des groupes N-protégés ou O-protégés, on déprotège et on soumet éventuellement le produit ainsi obtenu à une acylation ou alkylation pour obtenir les composés selon l'invention pour lesquels les substituants R₁, Rivet Rᵥ sont tels que définis pour (1), ou un de leurs sels.

5. Procédé de préparation des composés selon l'une des revendications 1 à 3, de formule (I) dans laquelle R est H, caractérisé en ce que l'on prépare le composé correspondant de formule I dans laquelle R représente H par acylation de l'aminothiazole avec une forme activée de l'acide de formule : 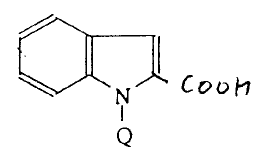 dans laquelle Q représente un groupe protecteur, pour obtenir le dérivé indolyle correspondant, après avoir déprotégé l'azote.

6. Composé de formule : 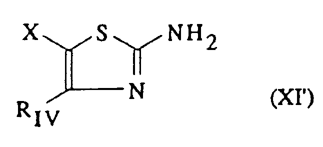 dans lequel R_{IV} est tel que défini dans la revendication 1 et X représente un alcoxy en C₁-C₅, une pipéridine non substituée ou substituée par un 4-hydroxy ou une 4-alcoxycarbonylpipérazine dans laquelle l'alcoxy est en C₁-C₃.

7. Composé de formule : 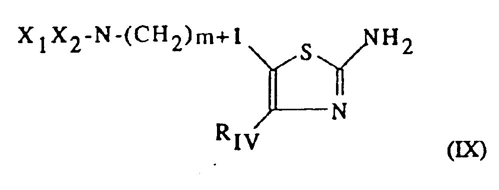 dans laquelle R_{IV} est tel que défini dans la revendication 1, m est 1 ou 2 et X₁X₂-N représente un groupe phtalimido ou -NH₂ ou un de ses sels.

8. Composés selon la revendication 1 de formule (I) dans laquelle Rᵥ représente -(CH₂)ₘ-X où X est un atome de brome.

## Claims (Claims for the following Contracting State(s) : AT, BE, CH, DE, DK, FR, GB, IT, LI, LU, NL, PT, SE)

1. 2-Acylamino thiazoles of the formula: 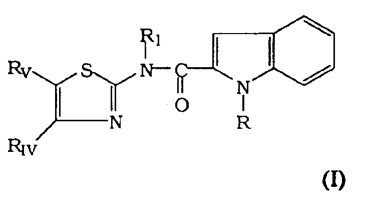 wherein
- R₁ denotes a hydrogen atom; a C₁_₄-alkyl group or an aminoalkylene group of formula -Z₁-NR₄R₅ wherein Z₁ denotes a C₂_₄-alkylene and R₄ and R₅ independently represent H or a C₁_₄-alkyl;
- R_{IV} represents a phenyl group which may be unsubstituted or carries one or more substituents selected from the halogen atoms, the C₁_₆-alkyl groups and C₁_₃-alkoxy groups or R_{IV} and R_{V} together represent the group: 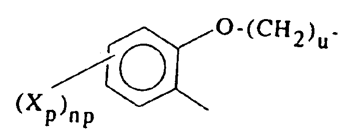 fixed by the carbon of the phenyl in the 4-position of the thiazolyl nucleus and wherein u represents 1 to 3, optionally carrying one or more (np) substituents Xp, which may be identical or different, selected from among the halogen atoms, the C₁_₃-alkyl and alkoxy groups, the nitro and trifluoromethyl groups, where np represents from 0 to 3;
Rᵥ denotes a -(CH₂)ₘ-X group wherein m is 0 to 5 and X denotes
a halogen atom, a hydroxyl, a C₃₋₇-cycloalkyl, a phenyl which may be substituted by one of the groups selected from among the halogen atoms, the C₁₋₃-alkyl or alkoxy groups, the nitro, amino, hydroxy or trifluoromethyl groups;
a group selected from among -COOH; -COOXᵢ; -O-COX₁; -SCOX₁; (O)_{q}-S-X₁ wherein q = ₀, 1 or 2; -O-COOX₁ ; 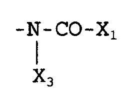 wherein X₃ = H or C₁_₃-alkyl; 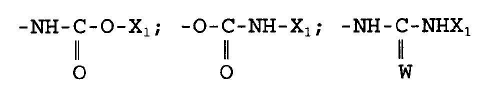 wherein W = O or S 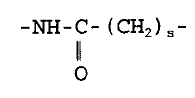 COOH wherein s = 2,3,4 wherein X₁ denotes a C₁_₅-alkyl; a phenyl which may be substituted by one or more groups selected from among the halogen atoms, the C₁_₃-alkyl or alkoxy groups, nitro, amino, hydroxy or trifluoromethyl; an adamantyl group;
a group selected from among -CONX₁X₂ and -NX₁X₂;
wherein X₁ denotes hydrogen, a C₁_₃-alkyl or a phenyl which may be unsubstituted or substituted by one or more groups selected from the halogen atoms, the C₁_₃-alkyl or alkoxy groups, nitro, amino, hydroxy and trifluoromethyl and X₂ denotes a hydrogen atom, a C₁₋₃-alkyl or X₁ and X₂ together with the nitrogen atom to which they are attached denote a heterocyclic selected from pyrrolidine or piperidine which may be unsubstituted or substituted by an oxo group or by a hydroxyl group, whilst the latter may be unsubstituted or substituted by an acyl or by a -COOX₁ or -CONX₁X₂ group;
or again Rᵥ denotes a C₁_₅-alkoxy; a hydroxyl group;
a 5- or 6-membered cyclic amine which may be unsubstituted or substituted by an oxo group or by a hydroxyl group; a piperazinyl group which may be unsubstituted or N-substituted by a group -COOAlk wherein Alk denotes a C₁_₅-alkyl; a carboxylic acid group, an -NX₂X₄ group wherein X₄ = H or X₄ = -(CH₂)ₜ-X₅, where t is 2, 3 or 4 and X₅ denotes -OH, -O-CO-R₂, -NHCOR₂, 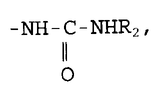 wherein R₂ denotes a C₁_₆-alkyl; or an -NR₂R₃ group wherein R₂ or R₃ independently represent H, C₁_₆-alkyl, a phenyl group which may be unsubstituted or substituted by one or more substituents selected from among the halogen atoms, a C₁_₃-alkyl group, a C₁_₃-alkoxy group or R₂ and R₃ together with the nitrogen atom to which they are attached denote a 5- or 6-membered heterocyclic group;
- R denotes H, a C₁_₄-alkyl, carboxyalkylene -Z₄-COOR₁₀ wherein Z₄ denotes (C₁_₄)alkylene and R₁₀ is H, benzyl or C₁_₆-alkyl; carbamoylalkylene -Z₅-CONR₁₁ R12 wherein Z₅ denotes (C₁_₄)alkylene and R₁₁ and R₁₂ independently denote H, (C₁_₆)alkyl or together with N form a saturated heterocyclic group such as morpholino or piperidino; acyl COR₁₃ wherein R₁₃ denotes (C₁_₄)alkyl or phenyl; alkoxycarbonyl -COOR₁₄ wherein R₁₄ denotes tert-butyl or benzyl;
and the addition salts of the compounds of formula (I) with inorganic or organic acids and bases.

2. Compounds according to claim 1 of formula (I) wherein, when R_{IV} denotes an optionally substituted phenyl group, the halogen atom or atoms is or are selected from fluorine and chlorine.

3. Compounds according to claim 1 of formula (I) wherein R_{IV} denotes a phenyl.

4. Process for preparing substituted 2-acylamino-5-thiazoles according to claim 1, characterised in that a substituted 2-amino-5-thiazole of formula: 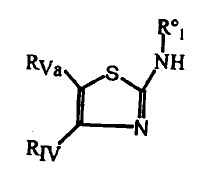 wherein R°₁ denotes the same substituents as R₁ in which the amino group present is N-protected, Rᵥₐ denotes the same substituents as Rᵥ wherein the hydroxy or amino groups are 0- and N-protected, is treated with a functional derivative of the acid of formula: 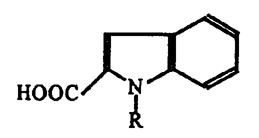 wherein the group NH is N-protected, then,
- when R°₁ or R_{Va} contain N-protected or O-protected groups, these groups are deprotected and the product thus obtained is optionally subjected to acylation or alkylation in order to obtain the compounds according to the invention wherein the substituents R₁, R_{IV} and R_{V} are as defined for (I) or a salt thereof.

5. Process for preparing compounds according to one of claims 1 to 3 of formula (I) wherein R is H, characterised in that the corresponding compound of formula (I) wherein R denotes H is prepared by acylating the aminothiazole with an activated form of the acid of formula: 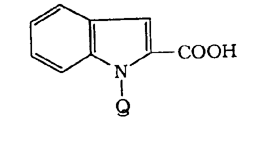 wherein Q denotes a protecting group, in order to obtain the corresponding indolyl derivative, after the nitrogen has been deprotected.

6. Compound of formula: 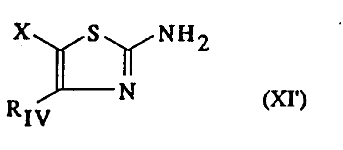 wherein R_{IV} is as defined in claim 1 and X denotes a C₁₋₅-alkoxy, a piperidine which is unsubstituted or substituted by a 4-hydroxy or 4-alkoxycarbonylpiperazine wherein the alkoxy has 1 to 3 carbon atoms.

7. Compound of formula: 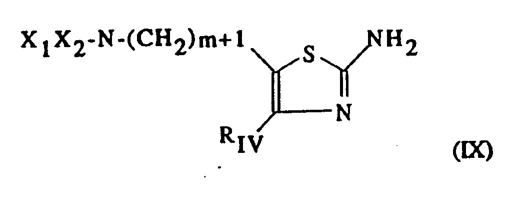 wherein R_{lV} is as defined in claim 1, m is 1 or 2 and XₗX₂-N denotes a phthalimido or -NH₂ group or a salt thereof.

8. Pharmaceutical composition, characterised in that it contains at least one compound according to one of claims 1 to 3 combined with at least one excipient.

9. Compounds according to claim 1 of formula (I) wherein R_{V} denotes -(CH₂)ₘ-X where X is a bromine atom.

## Claims (Claims for the following Contracting State(s) : ES)

1. Process for preparing 2-acylamino thiazoles of formula: 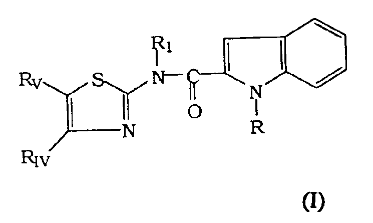 wherein
- R₁ denotes a hydrogen atom; a C₁_₄-alkyl group or an aminoalkylene group of formula -Z₁-NR₄R₅ wherein Z₁ denotes a C₂_₄-alkylene and R₄ and R₅ independently represent H or a C₁_₄-alkyl;
- R_{IV} represents a phenyl group which may be unsubstituted or carries one or more substituents selected from the halogen atoms, the C₁₋₆-alkyl groups and C₁_₃-alkoxy groups or R_{lV} and R_{V} together represent the group: 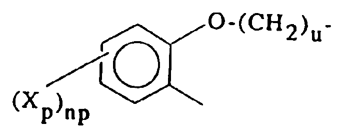 fixed by the carbon of the phenyl in the 4-position of the thiazolyl nucleus and wherein u represents 1 to 3, optionally carrying one or more (np) substituents Xp, which may be identical or different, selected from among the halogen atoms, the C₁_₃-alkyl and alkoxy groups, the nitro and trifluoromethyl groups, where np represents from 0 to 3;
- R_{V} denotes a -(CH₂)ₘ-X group wherein m is 0 to 5 and X denotes
a halogen atom, a hydroxyl, a C₃₋₇-cycloalkyl, a phenyl which may be substituted by one of the groups selected from among the halogen atoms, the C₁_₃-alkyl or alkoxy groups, the nitro, amino, hydroxy or trifluoromethyl groups;
a group selected from among -COOH; -COOX₁; -O-COX₁; -SCOX₁; (O)_{q}-S-X₁
wherein q = 0, 1 or 2; -O-COOX₁; 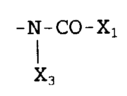 wherein X₃ = H or Cₗ-₃-alkyl; 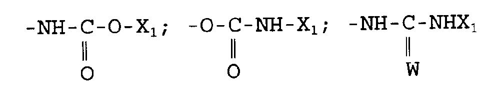 wherein W = O or S 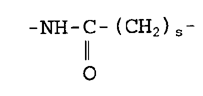 COOH wherein s = 2,3,4 wherein X₁ denotes a C₁_₅-alkyl; a phenyl which may be substituted by one or more groups selected from among the halogen atoms, the C₁_₃-alkyl or alkoxy groups, nitro, amino, hydroxy or trifluoromethyl; an adamantyl group;
a group selected from among -CONX₁X₂ and -NX₁X₂;
wherein X₁ denotes hydrogen, a C₁₋₃-alkyl or a phenyl which may be unsubstituted or substituted by one or more groups selected from the halogen atoms, the C₁_₃-alkyl or alkoxy groups, nitro, amino, hydroxy and trifluoromethyl and X₂ denotes a hydrogen atom, a C₁_₃-alkyl or X₁ and X₂ together with the nitrogen atom to which they are attached denote a heterocyclic selected from pyrrolidine or piperidine which may be unsubstituted or substituted by an oxo group or by a hydroxyl group, whilst the latter may be unsubstituted or substituted by an acyl or by a -COOX₁ or -CONX₁X₂ group;
or again Rᵥ denotes a C₁_₅-alkoxy; a hydroxyl group;
a 5- or 6-membered cyclic amine which may be unsubstituted or substituted by an oxo group or by a hydroxyl group; a piperazinyl group which may be unsubstituted or N-substituted by a group -COOAlk wherein Alkdenotes a C₁_₅-alkyl; a carboxylic acid group, an -NX₂X₄ group wherein X₄ = H or X₄ = -(CH₂)t⁻ X₅, where t is 2, 3 or 4 and X₅ denotes 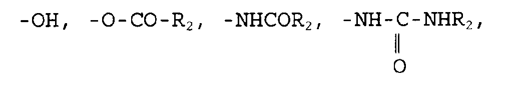 wherein R₂ denotes a C₁_₆-alkyl; or an -NR₂R₃ group wherein R₂ or R₃ independently represent H, C₁_₆-alkyl, a phenyl group which may be unsubstituted or substituted by one or more substituents selected from among the halogen atoms, a C₁_₃-alkyl group, a C₁₋₃-alkoxy group or R₂ and R₃ together with the nitrogen atom to which they are attached denote a 5- or 6-membered heterocyclic group;
- R denotes H, a C₁₋₄-alkyl, carboxyalkylene -Z₄-COOR₁₀ wherein Z₄ denotes (C₁_₄)alkylene and R₁₀ is H, benzyl or C₁₋₆-alkyl; carbamoylalkylene -Z₅-CONR₁ R12 wherein Z₅ denotes (C₁₋₄)alkylene and R₁ and R₁₂ independently denote H, (C₁_₆)alkyl or together with N form a saturated heterocyclic group such as morpholino or piperidino; acyl COR₁₃ wherein R₁₃ denotes (C₁_₄)alkyl or phenyl; alkoxycarbonyl -COOR₁₄ wherein R₁₄ denotes tert-butyl or benzyl;
and the addition salts of the compounds of formula (I) with inorganic or organic acids and bases, characterised in that a substituted 2-amino-5-thiazole of formula: 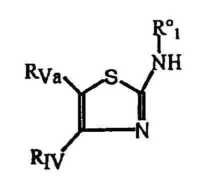 wherein R°₁ denotes the same substituents as R₁ in which the amino group present is N-protected, Rᵥₐ denotes the same substituents as Rᵥ wherein the hydroxy or amino groups are O- and N-protected, is treated with a functional derivative of the acid of formula: 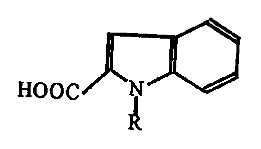 wherein the group NH is N-protected, then,
- when R°₁ or R_{Va} contain N-protected or 0-protected groups, these groups are deprotected and the product thus obtained is optionally subjected to acylation or alkylation in order to obtain the compounds according to the invention wherein the substituents R₁, R_{IV} and Rᵥ are as defined for (I) or a salt thereof.

2. Process according to claim 1, characterised in that, in formula (I), when Rᵢᵥ denotes optionally substituted phenyl, the halogen atom or atoms is or or selected from fluorine and chlorine.

3. Process according to claim 1, characterised in that in formula (I) R_{IV} denotes a phenyl.

4. Process for preparing compounds according to claim 3 of formula (I) wherein Z denotes the indole nucleus which is unsubstituted at the nitrogen and optionally substituted at the phenyl, characterised in that the corresponding compound of formula (I) wherein R denotes H is prepared by acylating the aminothiazole with an activated form of the acid of formula: 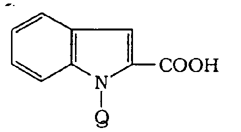 wherein Q represents a protecting group, to obtain the corresponding indolyl derivative, after the nitrogen has been deprotected.

## Claims (Claims for the following Contracting State(s) : GR)

1. 2-Acylamino thiazoles of the formula: 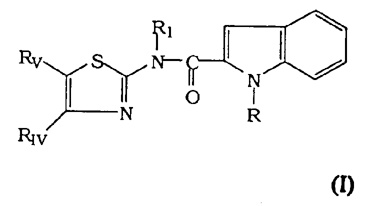 wherein
- R₁ denotes a hydrogen atom; a C₁₋₄-alkyl group or an aminoalkylene group of formula -Z₁-NR₄R₅ wherein Z₁ denotes a C₂_₄-alkylene and R₄ and R₅ independently represent H or a C₁_₄-alkyl;
- R_{IV} represents a phenyl group which may be unsubstituted or carries one or more substituents selected from the halogen atoms, the C₁_₆-alkyl groups and C₁_₃-alkoxy groups or R_{lV} and R_{V} together represent the group: 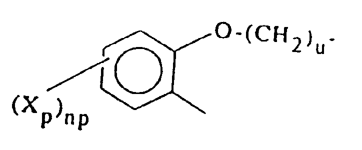 fixed by the carbon of the phenyl in the 4-position of the thiazolyl nucleus and wherein u represents 1 to 3, optionally carrying one or more (np) substituents Xp, which may be identical or different, selected from among the halogen atoms, the C₁_₃-alkyl and alkoxy groups, the nitro and trifluoromethyl groups, where np represents from 0 to 3;
R_{V} denotes a -(CH₂)ₘ-X group wherein m is 0 to 5 and X denotes
a halogen atom, a hydroxyl, a C₃₋₇-cycloalkyl, a phenyl which may be substituted by one of the groups selected from among the halogen atoms, the C₁_₃-alkyl or alkoxy groups, the nitro, amino, hydroxy or trifluoromethyl groups;
a group selected from among -COOH; -COOX₁; -O-COX₁; -SCOX₁; (O)_{q}-S-X₁ wherein q = 0, 1 or 2; -O-COOX₁; 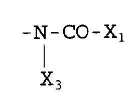 wherein X₃ = H or C₁_₃-alkyl; 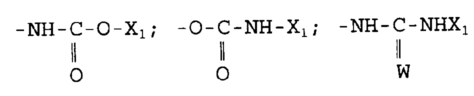 wherein W = 0 or S 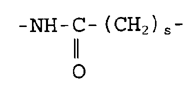 COOH wherein s = 2,3,4
wherein X₁ denotes a C₁_₅-alkyl; a phenyl which may be substituted by one or more groups selected from among the halogen atoms, the C₁_₃-alkyl or alkoxy groups, nitro, amino, hydroxy or trifluoromethyl;
an adamantyl group;
a group selected from among -CONX₁X₂ and -NX₁X₂;
wherein X₁ denotes hydrogen, a C₁_₃-alkyl or a phenyl which may be unsubstituted or substituted by one or more groups selected from the halogen atoms, the C₁_₃-alkyl or alkoxy groups, nitro, amino, hydroxy and trifluoromethyl and X₂ denotes a hydrogen atom, a C₁_₃-alkyl or X₁ and X₂ together with the nitrogen atom to which they are attached denote a heterocyclic group selected from pyrrolidine or piperidine which may be unsubstituted or substituted by an oxo group or by a hydroxyl group, whilst the latter may be unsubstituted or substituted by an acyl or by a -COOX₁ or -CONX₁X₂ group;
or again Rᵥ denotes a C₁_₅-alkoxy; a hydroxyl group;
a 5- or 6-membered cyclic amine which may be unsubstituted or substituted by an oxo group or by a hydroxyl group; a piperazinyl group which may be unsubstituted or N-substituted by a group -COOAlk wherein Alkdenotes a C₁_₅-alkyl; a carboxylic acid group, an -NX₂X₄ group wherein X₄ = H or X₄ = -(CH₂)ₜ-X₅, where t is 2, 3 or 4 and X₅ denotes -OH, -O-CO-R₂, -NHCOR_{z}, 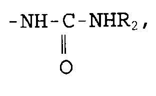 wherein R₂ denotes a C₁_₆-alkyl; or an -NR₂R₃ group wherein R₂ or R₃ independently represent H, C₁_₆-alkyl, a phenyl group which may be unsubstituted or substituted by one or more substituents selected from among the halogen atoms, a C₁_₃-alkyl group, a C₁_₃-alkoxy group or R₂ and R₃ together with the nitrogen atom to which they are attached denote a 5- or 6-membered heterocyclic group;
- R denotes H, a C₁₋₄-alkyl, carboxyalkylene -Z₄-COOR₁₀ wherein Z₄ denotes (C₁_₄)alkylene and R₁₀ is H, benzyl or C₁_₆-alkyl; carbamoylalkylene -Z₅-CONR₁₁ R₁₂ wherein Z₅ denotes (C₁-₄)alkylene and R₁₁ and R₁₂ independently denote H, (C₁_₆)alkyl or together with N form a saturated heterocyclic group such as morpholino or piperidino; acyl COR₁₃ wherein R₁₃ denotes (C₁_₄)alkyl or phenyl; alkoxycarbonyl -COOR₁₄ wherein R₁₄ denotes tert-butyl or benzyl;
and the addition salts of the compounds of formula (I) with inorganic or organic acids and bases.

2. Compounds according to claim 1 of formula (I) wherein, when R_{IV} denotes an optionally substituted phenyl group, the halogen atom or atoms is or are selected from fluorine and chlorine.

3. Compounds according to claim 1 of formula (I) wherein R_{lV} denotes a phenyl.

4. Process for preparing substituted 2-acylamino-5-thiazoles according to claim 1, characterised in that a substituted 2-amino-5-thiazole of formula: 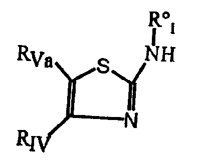 wherein R°₁ denotes the same substituents as R₁ in which the amino group present is N-protected, Rᵥₐ denotes the same substituents as Rᵥ wherein the hydroxy or amino groups are 0- and N-protected, is treated with a functional derivative of the acid of formula: 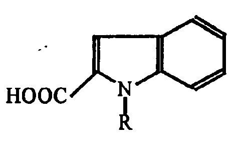 wherein the group NH is N-protected, then,
- when R°₁ or R_{Va} contain N-protected or 0-protected groups, these groups are deprotected and the product thus obtained is optionally subjected to acylation or alkylation in order to obtain the compounds according to the invention wherein the substituents R₁, R_{IV} and Rᵥ are as defined for (I) or a salt thereof.

5. Process for preparing compounds according to one of claims 1 to 3 of formula (I) wherein R is H, characterised in that the corresponding compound of formula (I) wherein R denotes H is prepared by acylating the aminothiazole with an activated form of the acid of formula: 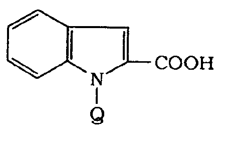 wherein Q denotes a protecting group, in order to obtain the corresponding indolyl derivative, after the nitrogen has been deprotected.

6. Compound of formula: 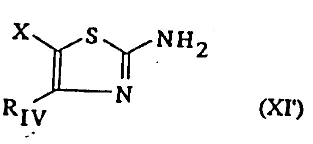 wherein R_{IV} is as defined in claim 1 and X denotes a C₁_₅-alkoxy, a piperidine which is unsubstituted or substituted by a 4-hydroxy or 4-alkoxycarbonylpiperazine wherein the alkoxy has 1 to 3 carbon atoms.

7. Compound of formula: 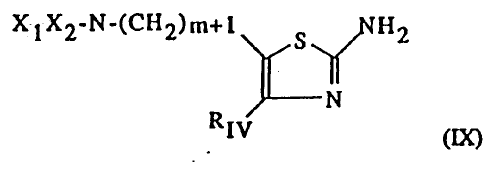 wherein R_{lV} is as defined in claim 1, m is 1 or 2 and X₁X₂-N denotes a phthalimido or -NH₂ group or a salt thereof.

8. Compounds according to claim 1 of formula (I) wherein Rᵥ denotes -(CH₂)ₘ-X where X is a bromine atom.

## Patentansprüche (Patentansprüche für folgende(n) Vertragsstaat(en) : AT, BE, CH, DE, DK, FR, GB, IT, LI, LU, NL, PT, SE)

1. 2-Acylamino-thiazole der Formel: 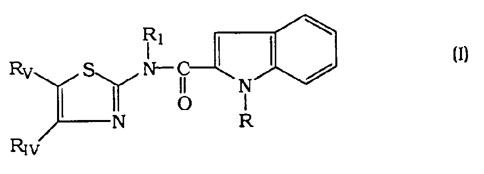 in der
- R₁ ein Wasserstoffatom, eine C₁-C₄-Alkylgruppe oder eine Aminoalkylengruppe der Formel-Z₁-NR₄R₅, in der Z₁ eine C₂-C₄-Alkylengruppe und R₄ und R₅ unabhängig voneinander H oder eine C₁-C₄-Alkylgruppe darstellen;
- R_{IV} eine Phenylgruppe, die unsubstituiert ist oder einen oder mehrere Substituenten ausgewählt aus Halogenatomen, (C₁-C₆)-Alkylgruppen und C₁-C₃-Alkoxygruppen trägt; oder R_{lV} und Rᵥ gemeinsam die Gruppe: 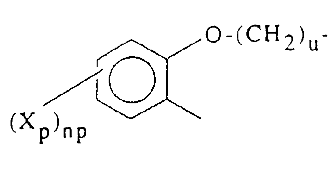 die über das Kohlenstoffatom des Phenylrests in der 4-Stellung des Thiazolylkerns gebunden ist und in der u den Wert 1 bis 3 besitzt, welche gegebenenfalls einen oder mehrere (np) Substituenten Xp trägt, die gleichartig oder verschieden sind und aus Halogenatomen, C₁-C₃-Alkylgruppen, C₁-C₃-Alkoxygruppen, Nitrogruppen und Trifluormethylgruppen ausgewählt sind und np einen Wert von 0 bis 3 besitzt;
Rᵥ eine Gruppe -(CH₂)ₘ-X, in der m 0 bis 5 und X
ein Halogenatom, eine Hydroxylgruppe, eine C₃-C₇-Cycloalkylgruppe, eine Phenylgruppe, die durch eine der Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann;
eine Gruppe ausgewählt aus -COOH; -COOX₁; -O-COX₁; -SCOX₁; (0)q-S-Xᵢ, worin q = 0, 1 oder 2 darstellt; 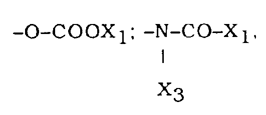 worin X₃ = H oder eine C₁-C₃-Alkylgruppe darstellt; 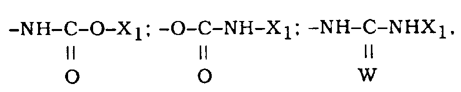 worin W = O oder S darstellt; 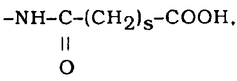 worin s 2, 3 oder 4 darstellt
und X₁ eine C₁-C₅-Alkylgruppe; eine Phenylgruppe, die durch eine oder mehrere Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann; oder eine Adamantylgruppe darstellt;
eine Gruppe ausgewählt aus -CONX₁X₂; -NX₁X₂; worin X₁ Wasserstoff, eine C₁-C₃-Alkylgruppe oder eine Phenylgruppe, die unsubstituiert oder durch eine oder mehrere Gruppen ausgewählt aus Halogenatomen, Cᵢ-C₃-Alkylgruppen, C₁-C₃-Alkoxygruppen, Nitrogruppen, Aminogruppen, Hydroxygruppen, Trifluormethylgruppen substituiert ist, und X₂ ein Wasserstoffatom, eine C₁-C₃-Alkylgruppe oder X₁ und X₂ zusammen mit dem Stickstoffatom, an das sie gebunden sind, einen Heterocyclus ausgewählt aus Pyrrolidin oder Piperidin, der nicht substituiert oder durch eine Oxogruppe oder eine Hydroxylgruppe substituiert ist, wobei die letztere gegebenenfalls durch eine Acylgruppe, eine Gruppe -COOX₁ oder -CONX₁X₂ substituiert sein kann, darstellen;
oder R_{V} eine C₁-C₅-Alkoxygruppe; eine Hydroxylgruppe; eine cyclische Amingruppe mit 5 oder 6 Kettengliedern, die unsubstituiert ist oder durch eine Oxogruppe oder eine Hydroxylgruppe substituiert ist; eine Piperazinylgruppe, die unsubstituiert ist oder durch eine Gruppe -COOAlk, worin Alk eine C₁-C₅-Alkylgruppe darstellt, N-substituiert ist; eine Carbonsäuregruppe, eine Gruppe -NX₂X₄, in der X₄ = H oder X₄ = -(CH₂)ₜ-X₅, worin t 2, 3 oder 4 und X₅ -OH, -O-CO-R₂, -NHCOR₂, 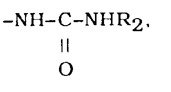
worin R₂ für eine C₁-C₆-Alkylgruppe steht, darstellen; eine Gruppe -NR₂R₃, worin R₂ oder R₃ unabhängig voneinander H, C₁-C₆-Alkyl, eine Phenylgruppe, die unsubstituiert oder durch einen oder mehrere Substituenten ausgewählt aus Halogenatomen, C₁-C₃-Alkylgruppen, C₁-C₃-Alkoxygruppen substituiert ist oder R₂ und R₃ zusammen mit dem Stickstoffatom, an das sie gebunden sind, einen Heterocyclus mit 5 oder 6 Kettengliedern bilden; und
- R H, (C₁-C₄)-Alkyl, Carbalkoxyalkylen -Z₄-COOR₁₀, worin Z₄ (C₁-C₄)-Alkylen und R₁₀ H, Benzyl oder (C₁-C₆)-Alkyl darstellen; Carbamoylalkylen -Z₅-COONR_{I1}R₁₂, worin Z5 (C₁-C₄)-Alkylen und R₁₁ und R₁₂ unabhängig voneinander H, (C₁-C₆)-Alkyl oder zusammen mit N einen gesättigten Heterocyclus, wie Morpholino oder Piperidino, bilden; Acyl -COR₁₃, worin R₁₃ (C₁-C₄)-Alkyl oder Phenyl darstellt; Alkoxycarbonyl -COOR₁₄, worin R₁₄ tert.-Butyl oder Benzyl darstellt, bedeuten;
sowie die Additionssalze der Verbindungen der Formel (I) mit anorganischen oder organischen Säuren oder Basen.

2. Verbindungen nach Anspruch 1 der Formel (I), in der Rₗᵥ eine gegebenenfalls substituierte Phenylgruppe darstellt und das oder die Halogenatome aus Fluor und Chlor ausgewählt sind.

3. Verbindungen nach Anspruch 1 der Formel (I), in der R_{IV} eine Phenylgruppe darstellt.

4. Verfahren zur Herstellung der substituierten 2-Acylamino-5-thiazole nach Anspruch 1, dadurch gekennzeichnet, daß man ein substituiertes 2-Amino-5-thiazol der Formel: 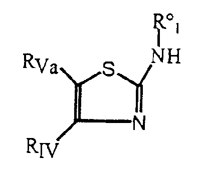 in der R^{O}₁ die gleichen Substituenten darstellt wie R₁, in der die vorhandene Aminogruppe N-geschützt ist und Rᵥₐ die gleichen Substituenten bedeutet wie R_{V}, worin die Hydroxygruppen oder Aminogruppen O- und N-geschützt sind, mit einem funktionellen Säurederivat der Formel: 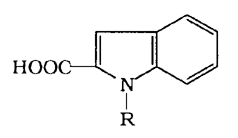 in der die NH-Gruppe N-geschützt ist, umsetzt und dann,
- wenn R°₁ oder R_{Va} N-geschützte oder O-geschützte Gruppen enthalten, man die Schutzgruppen abspaltet und das in dieser Weise erhaltene Produkt gegebenenfalls einer Acylierung oder Alkylierung unterwirft zur Bildung der erfindungsgemäßen Verbindungen, bei denen die Substituenten R₁, R_{IV} und R_{V} die bezüglich der Formel (I) angegebenen Bedeutungen besitzen, oder eines ihrer Salze.

5. Verfahren zur Herstellung der Verbindungen nach einem der Ansprüche 1 bis 3 der Formel (I), in der R H bedeutet, dadurch gekennzeichnet, daß man die entsprechende Verbindung der Formel herstellt, in der R H bedeutet, durch Acylieren von Aminothiazol mit einer aktiven Form der Säure der Formel: 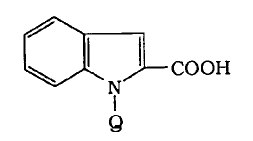 in der Q eine Schutzgruppe darstellt, so daß man nach der Abspaltung der Schutzgruppe von dem Stickstoff das entsprechende Indolyl-Derivat erhält.

6. Verbindung der Formel: 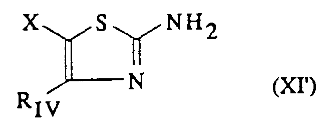 in der R_{IV} die in Anspruch 1 angegebenen Bedeutungen besitzt und X eine C₁-C₅-Alkoxygruppe, eine Piperidingruppe, die unsubstituiert oder durch eine 4-Hydroxygruppe substituiert ist, oder eine 4-Alkoxycarbonylpiperazingruppe, in der die Alkoxygruppe eine C₁-C₃-Gruppe ist, bedeuten.

7. Verbindung der Formel: 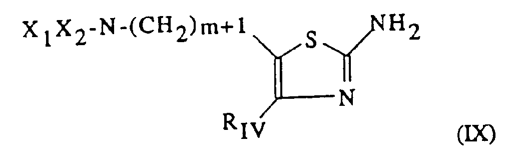 in der R_{IV} die in Anspruch 1 angegebenen Bedeutungen besitzt, m 1 oder 2 darstellt und X₁X₂-N eine Phthalimidogruppe oder eine Gruppe -NH₂ bedeuten, sowie ihre Salze.

8. Pharmazeutische Zubereitung, dadurch gekennzeichnet, daß sie mindestens eine Verbindung nach einem der Ansprüche 1 bis 3 in Kombination mit mindestens einem Trägermaterial enthält.

9. Verbindungen nach Anspruch 1 der Formel (I), In der R_{V}-(CH₂)ₘ-X darstellt, in der X ein Bromatom bedeutet.

## Patentansprüche (Patentansprüche für folgende(n) Vertragsstaat(en) : ES)

1. Verfahren zur Herstellung von 2-Acylamino-thiazolen der Formel: 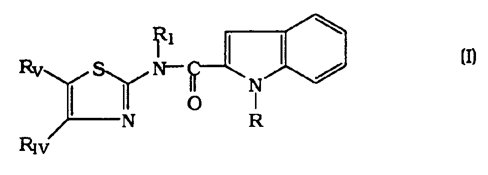 in der
- R₁ ein Wasserstoffatom, eine C₁-C₄-Alkylgruppe oder eine Aminoalkylengruppe der Formel -Z₁-NR₄R₅, in der Z₁ eine C₂-C₄-Alkylengruppe und R₄ und R₅ unabhängig voneinander H oder eine C₁-C₄-Alkylgruppe darstellen;
- R_{IV} eine Phenylgruppe, die unsubstituiert ist oder einen oder mehrere Substituenten ausgewählt aus Halogenatomen, (C₁-C₆)-Alkylgruppen und C₁-C₃-Alkoxygruppen trägt; oder R_{IV} und Rᵥ gemeinsam die Gruppe: 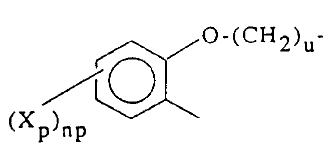 die über das Kohlenstoffatom des Phenylrests in der 4-Stellung des Thiazolylkerns gebunden ist und in der u den Wert 1 bis 3 besitzt, welche gegebenenfalls einen oder mehrere (np) Substituenten Xp trägt, die gleichartig oder verschieden sind und aus Halogenatomen, C₁-C₃-Alkylgruppen, C₁-C₃-Alkoxygruppen, Nitrogruppen und Trifluormethylgruppen ausgewählt sind und np einen Wert von 0 bis 3 besitzt;
R_{V} eine Gruppe -(CH₂)ₘ-X, in der m 0 bis 5 und X
ein Halogenatom, eine Hydroxylgruppe, eine C₃-C₇-Cycloalkylgruppe, eine Phenylgruppe, die durch eine der Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann;
eine Gruppe ausgewählt aus -COOH; -COOX₁; -O-COX₁; -SCOX₁; (O)q-S-X₁, worin q = 0, oder 2 darstellt; 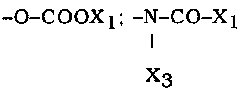 worin X₃ = H oder eine C₁-C₃-Alkylgruppe darstellt: 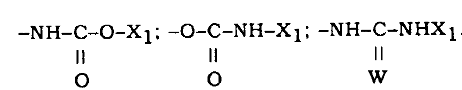 , worin W = O oder S darstellt: 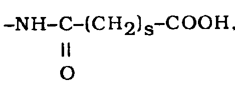 worin s 2. 3 oder 4 darstellt
und X₁ eine C₁-C₅-Alkylgruppe; eine Phenylgruppe, die durch eine oder mehrere Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann; oder eine Adamantylgruppe darstellt; . eine Gruppe ausgewählt aus -CONX₁X₂; -NX₁X₂; worin X₁ Wasserstoff, eine C₁-C₃-Alkylgruppe oder eine Phenylgruppe, die unsubstituiert oder durch eine oder mehrere Gruppen ausgewählt aus Halogenatomen, C₁-C₃-Alkylgruppen, C₁-C₃-Alkoxygruppen, Nitrogruppen, Aminogruppen, Hydroxygruppen, Trifluormethylgruppen substituiert ist, und X₂ ein Wasserstoffatom, eine C₁-C₃-Alkylgruppe oder X₁ und X₂ zusammen mit dem Stickstoffatom, an das sie gebunden sind, einen Heterocyclus ausgewählt aus Pyrrolidin oder Piperidin, der nicht substituiert oder durch eine Oxogruppe oder eine Hydroxylgruppe substituiert ist, wobei die letztere gegebenenfalls durch eine Acylgruppe, eine Gruppe -COOX₁ oder -CONX₁X2 substituiert sein kann, darstellen;
oder R_{V} eine C₁-C₅-Alkoxygruppe; eine Hydroxylgruppe; eine cyclische Amingruppe mit 5 oder 6 Kettengliedern, die unsubstituiert ist oder durch eine Oxogruppe oder eine Hydroxylgruppe substituiert ist; eine Piperazinylgruppe, die unsubstituiert ist oder durch eine Gruppe -COOAlk, worin Alk eine C₁-C₅-Alkylgruppe darstellt, N-substituiert ist; eine Carbonsäuregruppe, eine Gruppe -NX₂X₄, in der X₄ = H oder X₄ = -(CH₂)ₜ-X₅, worin t 2, 3 oder 4 und X₅ -OH, -O-CO-R₂, -NHCOR₂. 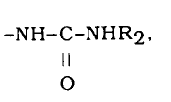
worin R₂ für eine C₁-C₆-Alkylgruppe steht, darstellen; eine Gruppe -NR₂R₃, worin R₂ oder R₃ unabhängig voneinander H, C₁-C₆-Alkyl, eine Phenylgruppe, die unsubstituiert oder durch einen oder mehrere Substituenten ausgewählt aus Halogenatomen, C₁-C₃-Alkylgruppen, C₁-C₃-Alkoxygruppen substituiert ist oder R₂ und R₃ zusammen mit dem Stickstoffatom, an das sie gebunden sind, einen Heterocyclus mit 5 oder 6 Kettengliedern bilden; und
- R H, (C₁-C₄)-Alkyl, Carbalkoxyalkylen -Z₄-COOR_{10'} worin Z₄ (C₁-C₄)-Alkylen und R₁₀ H, Benzyl oder (C₁-C₆)-Alkyl darstellen; Carbamoylalkylen -Z₅-COONR₁₁R₁₂, worin Z₅ (C₁-C₄)-Alkylen und R₁₁ und R₁₂ unabhängig voneinander H, (C₁-C₆)-Alkyl oder zusammen mit N einen gesättigten Heterocyclus, wie Morpholino oder Piperidino, bilden; Acyl -COR₁₃, worin R₁₃ (C₁-C₄)-Alkyl oder Phenyl darstellt; Alkoxycarbonyl -COOR₁₄, worin R₁₄ tert.-Butyl oder Benzyl darstellt, bedeuten;
sowie von den Additionssalzen der Verbindungen der Formel (I) mit anorganischen oder organischen Säuren oder Basen,
dadurch gekennzeichnet, daß man ein substituiertes 2-Amino-5-thiazol der Formel: 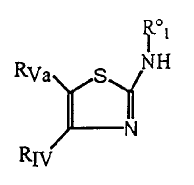 in der R°₁ die gleichen Substituenten darstellt wie R₁, in der die vorhandene Aminogruppe N-geschützt ist und R_{Va} die gleichen Substituenten bedeutet wie R_{V}, worin die Hydroxygruppen oder Aminogruppen 0- und N-geschützt sind, mit einem funktionellen Säurederivat der Formel: 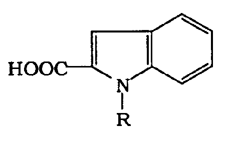 in der die NH-Gruppe N-geschützt ist, umsetzt und dann,
- wenn R°₁ oder R_{Va} N-geschützte oder O-geschützte Gruppen enthalten, man die Schutzgruppen abspaltet und das in dieser Weise erhaltene Produkt gegebenenfalls einer Acylierung oder Alkylierung unterwirft zur Bildung der erfindungsgemäßen Verbindungen, bei denen die Substituenten R_{1'} Rₗᵥ und Rᵥ die bezüglich der Formel (I) angegebenen Bedeutungen besitzen, oder eines ihrer Salze.

2. Verfahren nach Anspruch 1, dadurch gekennzeichnet, daß in der Formel (I), wenn Rₗᵥ eine gegebenenfalls substituierte Phenylgruppe darstellt, das oder die Halogenatome aus Fluor und Chlor ausgewählt sind.

3. Verfahren nach Anspruch 1, dadurch gekennzeichnet, daß in der Formel (I) R_{IV} eine Phenylgruppe bedeutet.

4. Verfahren zur Herstellung der Verbindungen nach Anspruch 3 der Formel (I), in der Z den Indolkern bedeutet, der am Stickstoffatom nicht substituiert und gegebenenfalls am Phenylrest substituiert ist, dadurch gekennzeichnet, daß man die entsprechende Verbindung der Formel (I) herstellt, in der R H bedeutet, durch Acylierung des Aminothiazols mit einer aktiven Form der Säure der Formel: 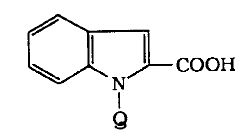 in der Q eine Schutzgruppe darstellt, so daß man nach der Abspaltung der Schutzgruppe vom Stickstoffatom das entsprechende Indolyl-Derivat erhält.

## Patentansprüche (Patentansprüche für folgende(n) Vertragsstaat(en) : GR)

1. 2-Acylamino-thiazole der Formel: 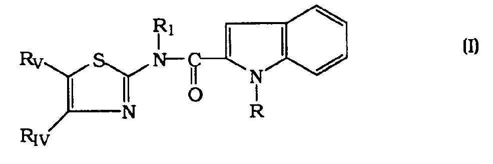 in der
- R₁ ein Wasserstoffatom, eine C₁-C₄-Alkylgruppe oder eine Aminoalkylengruppe der Formel-Z₁-NR₄R₅, in der Z₁ eine C₂-C₄-Alkylengruppe und R₄ und R₅ unabhängig voneinander H oder eine C₁-C₄-Alkylgruppe darstellen;
- R_{IV} eine Phenylgruppe, die unsubstituiert ist oder einen oder mehrere Substituenten ausgewählt aus Halogenatomen, (C₁-C₆)-Alkylgruppen und C₁-C₃-Alkoxygruppen trägt; oder R_{IV} und Rᵥ gemeinsam die Gruppe: 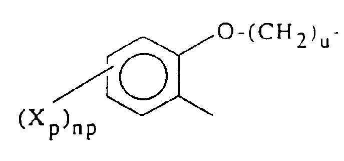 die über das Kohlenstoffatom des Phenylrests in der 4-Stellung des Thiazolylkerns gebunden ist und in der u den Wert 1 bis 3 besitzt, welche gegebenenfalls einen oder mehrere (np) Substituenten Xp trägt, die gleichartig oder verschieden sind und aus Halogenatomen, C₁-C₃-Alkylgruppen, C₁-C₃-Alkoxygruppen, Nitrogruppen und Trifluormethylgruppen ausgewählt sind und np einen Wert von 0 bis 3 besitzt;
- Rᵥ eine Gruppe -(CH₂)ₘ-X, in der m 0 bis 5 und X
ein Halogenatom, eine Hydroxylgruppe, eine C₃-C₇-Cycloalkylgruppe, eine Phenylgruppe, die durch eine der Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann;
eine Gruppe ausgewählt aus -COOH; -COOX₁; -O-COX₁; -SCOX₁; (O)_{q}-S-X₁, worin q = 0, oder 2 darstellt; 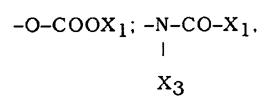 worin X₃ = H oder eine C₁-C₃-Alkylgruppe darstellt; 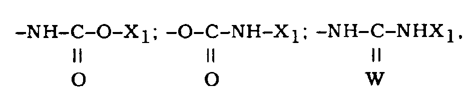 worin W = O oder S darstellt: 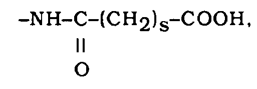 worin s 2. 3 oder 4 darstellt
und X₁ eine C₁-C₅-Alkylgruppe; eine Phenylgruppe, die durch eine oder mehrere Gruppen ausgewählt aus Halogenatomen, C₁-C₃-Alkylgruppen, C₁-C₃-Alkoxygruppen, Nitrogruppen, Aminogruppen, Hydroxygruppen oder Trifluormethylgruppen substituiert sein kann; oder eine Adamantylgruppe darstellt; . eine Gruppe ausgewählt aus -CONX₁X₂; -NX₁X₂; worin X₁ Wasserstoff, eine C₁-C₃-Alkylgruppe oder eine Phenylgruppe, die unsubstituiert oder durch eine oder mehrere Gruppen ausgewählt aus Halogenatomen, C₁-C₃-Alkylgruppen, C₁-C₃-Alkoxygruppen, Nitrogruppen, Aminogruppen, Hydroxygruppen, Trifluormethylgruppen substituiert ist, und X₂ ein Wasserstoffatom, eine C₁-C₃-Alkylgruppe oder X₁ und X₂ zusammen mit dem Stickstoffatom, an das sie gebunden sind, einen Heterocyclus ausgewählt aus Pyrrolidin oder Piperidin, der nicht substituiert oder durch eine Oxogruppe oder eine Hydroxylgruppe substituiert ist, wobei die letztere gegebenenfalls durch eine Acylgruppe, eine Gruppe -COOX₁ oder -CONX₁X₂ substituiert sein kann, darstellen;
oder R_{V} eine C₁-C₅-Alkoxygruppe; eine Hydroxylgruppe; eine cyclische Amingruppe mit 5 oder 6 Kettengliedern, die unsubstituiert ist oder durch eine Oxogruppe oder eine Hydroxylgruppe substituiert ist; eine Piperazinylgruppe, die unsubstituiert ist oder durch eine Gruppe -COOAlk, worin Alk eine C₁-C₅-Alkylgruppe darstellt, N-substituiert ist; eine Carbonsäuregruppe, eine Gruppe -NX₂X₄, in der X₄ = H oder X₄ = -(CH₂)ₜ-X₅, worin t 2, 3 oder 4 und X₅ -OH. -O-CO-R₂, -NHCOR₂, 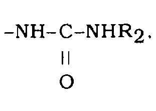
worin R₂ für eine C₁-C₆-Alkylgruppe steht, darstellen; eine Gruppe -NR₂R₃, worin R₂ oder R₃ unabhängig voneinander H, C₁-C₆-Alkyl, eine Phenylgruppe, die unsubstituiert oder durch einen oder mehrere Substituenten ausgewählt aus Halogenatomen, C₁-C₃-Alkylgruppen, C₁-C₃-Alkoxygruppen substituiert ist oder R₂ und R₃ zusammen mit dem Stickstoffatom, an das sie gebunden sind, einen Heterocyclus mit 5 oder 6 Kettengliedern bilden; und
- R H, (C₁-C₄)-Alkyl, Carbalkoxyalkylen -Z₄-COOR_{1O'} worin Z₄ (C₁-C₄)-Alkylen und R₁₀ H, Benzyl oder (C₁-C₆)-Alkyl darstellen; Carbamoylalkylen -Z₅-COONR₁₁R₁₂, worin Z5 (C₁-C₄)-Alkylen und R₁₁ und R₁₂ unabhängig voneinander H, (C₁-C₆)-Alkyl oder zusammen mit N einen gesättigten Heterocyclus, wie Morpholino oder Piperidino, bilden; Acyl -COR₁₃, worin R₁₃ (C₁-C₄)-Alkyl oder Phenyl darstellt; Alkoxycarbonyl -COOR₁₄, worin R₁₄ tert.-Butyl oder Benzyl darstellt, bedeuten;
sowie die Additionssalze der Verbindungen der Formel (I) mit anorganischen oder organischen Säuren oder Basen.

2. Verbindungen nach Anspruch 1 der Formel (I), in der R_{IV} eine gegebenenfalls substituierte Phenylgruppe darstellt und das oder die Halogenatome aus Fluor und Chlor ausgewählt sind.

3. Verbindungen nach Anspruch 1 der Formel (I), in der R_{IV} eine Phenylgruppe darstellt.

4. Verfahren zur Herstellung der substituierten 2-Acylamino-5-thiazole nach Anspruch 1, dadurch gekennzeichnet, daß man ein substituiertes 2-Amino-5-thiazol der Formel: 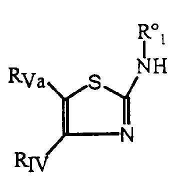 in der R°₁ die gleichen Substituenten darstellt wie R₁, in der die vorhandene Aminogruppe N-geschützt ist und Rᵥₐ die gleichen Substituenten bedeutet wie R_{V}, worin die Hydroxygruppen oder Aminogruppen O- und N-geschützt sind, mit einem funktionellen Säurederivat der Formel: 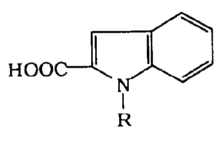 in der die NH-Gruppe N-geschützt ist, umsetzt und dann,
- wenn R^{O}₁ oder R_{Va} N-geschützte oder O-geschützte Gruppen enthalten, man die Schutzgruppen abspaltet und das in dieser Weise erhaltene Produkt gegebenenfalls einer Acylierung oder Alkylierung unterwirft zur Bildung der erfindungsgemäßen Verbindungen, bei denen die Substituenten R_{1'} R_{IV} und R_{V} die bezüglich der Formel (I) angegebenen Bedeutungen besitzen, oder eines ihrer Salze.

5. Verfahren zur Herstellung der Verbindungen nach einem der Ansprüche 1 bis 3 der Formel (I), in der R H bedeutet, dadurch gekennzeichnet, daß man die entsprechende Verbindung der Formel herstellt, in der R H bedeutet, durch Acylieren von Aminothiazol mit einer aktiven Form der Säure der Formel: 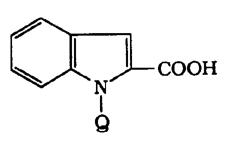 in der Q eine Schutzgruppe darstellt, so daß man nach der Abspaltung der Schutzgruppe von dem Stickstoff das entsprechende Indolyl-Derivat erhält.

6. Verbindung der Formel: 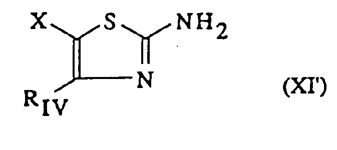 in der R_{IV} die in Anspruch 1 angegebenen Bedeutungen besitzt und X eine C₁-C₅-Alkoxygruppe, eine Piperidingruppe, die unsubstituiert oder durch eine 4-Hydroxygruppe substituiert ist, oder eine 4-Alkoxycarbonylpiperazingruppe, in der die Alkoxygruppe eine C₁-C₃-Gruppe ist, bedeuten.

7. Verbindung der Formel: 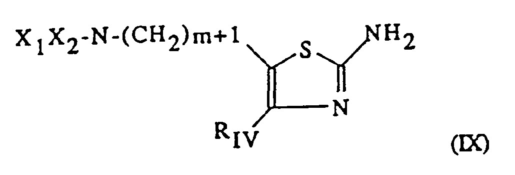 in der R_{IV} die in Anspruch 1 angegebenen Bedeutungen besitzt, m 1 oder 2 darstellt und X₁X₂-N eine Phthalimidogruppe oder eine Gruppe -NH₂ bedeuten, sowie ihre Salze.

8. Verbindungen nach Anspruch 1 der Formel (I), in der R_{V}-(CH₂)ₘ-X darstellt, worin X ein Bromatom bedeutet.
